# EUROPEAN PATENT SPECIFICATION

(11) **EP 3 548 896 B1**
(45) Date of publication and mention of the grant of the patent: **06.01.2021**
(21) Application number: 17808867.0
(22) Date of filing: 30.11.2017
(51) Int. Cl.: G01N 33/68

(54) **USE OF ANTI-UCHL1 IGG PLASMA CONCENTRATION FOR DIAGNOSING IDIOPATHIC STEROID SENSITIVE NEPHROTIC SYNDROME**
VERWENDUNG DER IGG PLASMAKONZENTRATION VON ANTI-UCHL1 ZUR DIAGNOSE VON IDIOPATHISCHEM STEROIDSENSITIVEM NEPHROTISCHEM SYNDROM
UTILISATION DE LA CONCENTRATION DE IGG CONTRE UCHL1 POUR DIAGNOSTIQUER LE SYNDROME NÉPHROTIQUE IDIOPATHIQUE SENSIBLE AUX STÉROÏDES

(30) Priority: 01.12.2016 EP 16306596; 18.01.2017 EP 17305055
(43) Date of publication of application: 09.10.2019
(73) Proprietor: INSERM (Institut National de la Santé et de la Recherche Médicale), 75013 Paris (FR); Assistance Publique-Hôpitaux de Paris, 75004 Paris (FR); Université Paris Diderot Paris 7, 75013 Paris (FR); Centre National de la Recherche Scientifique (CNRS), 75016 Paris (FR)
(72) Inventor: DESCHENES, Georges, 75870 Paris Cedex 18 (FR); JAMIN, Agnès, 35760 Saint Gregoire (FR); MONTEIRO, Renato, 75870 Paris Cedex 18 (FR)
(74) Representative: Inserm Transfert
(86) International application number: PCT/EP2017/081046
(87) International publication number: WO 2018/100096

(56) References cited:
- US-A1- 2011 212 079
- L. MUSANTE ET AL: "Circulating anti-actin and anti-ATP synthase antibodies identify a sub-set of patients with idiopathic nephrotic syndrome", CLINICAL AND EXPERIMENTAL IMMUNOLOGY, vol. 141, no. 3, 1 September 2005 (2005-09-01), pages 491-499, XP055357287, GB ISSN: 0009-9104, DOI: 10.1111/j.1365-2249.2005.02862.x
- "Abstracts of the 50th Anniversary ESPN Meeting, Glasgow, September 2017", PEDIATRIC NEPHROLOGY, SPRINGER VERLAG, BERLIN, DE, vol. 32, no. 9, 24 August 2017 (2017-08-24), pages 1643-1834, XP036306068, ISSN: 0931-041X, DOI: 10.1007/S00467-017-3753-X [retrieved on 2017-08-24]

## Description

### FIELD:

The present disclosure is in the field of medicine, in particular nephrology.

### BACKGROUND:

Idiopathic steroid sensitive nephrotic syndrome (INS) is the most frequent childhood nephropathy characterized by proteinuria and podocyte injury.¹ This pathology is one of the major chronic pediatric diseases with a long lasting course in 50 % of children. The dramatic effect of steroids to cancel proteinuria and the efficacy and immunosuppressive treatments to prevent relapses suggests that INS is not a kidney disease, but a primary immune disease.2

It is supposed that podocytes are the exclusive target of a circulating glomerular permeability factor that mediates foot process effacement and proteinuria.³ Despite extensive investigation, INS pathophysiology remains unclear and the soluble circulating factor has not been yet identified.

Nowadays, the increasing therapeutic experience as well as basic investigations in INS supports the hypothesis of a primary B cell disease. Calcineurin antagonists (cyclosporine and tacrolimus) widely used in INS to avoid steroid intoxication and to keep patients in remission⁴ are able to alter the differentiation of B cells in plasmablasts.⁵ Mycophenolate, another steroid-sparing agent in INS,⁶blocks the proliferation of either T or B lymphocytes and impairs antibody production.⁷ Rituximab is a monoclonal antibody raised against CD20, a specific surface marker of B cells that is not expressed by the plasma cells. The beneficial effect of rituximab in steroid dependent patients to spare relapses is certainly the most outstanding evidence of B cell involvement in INS.⁸ Moreover a high level of memory B cells, and especially of switched memory B cells, is predictive of a relapse after B cell repletion following rituximab therapy in steroid dependent INS.⁹ Consistently with the hypothesis of an abnormal subset of B cells producing an antibody directed against a renal target, immunoadsorption of plasma immunoglobulins is able to control the massive proteinuria in patients experiencing a recurrence of the disease after a renal graft.^{10, 11}

Musante L. et al. report circulating anti - actin/ATP synthase *β* chain antibodies in a subset of patients with INS (Musante, L., et al. "Circulating anti - actin and anti - ATP synthase antibodies identify a sub - set of patients with idiopathic nephrotic syndrome. " Clinical & Experimental Immunology 141.3 (2005): 491-499*).*

US20110212079 discloses methods for treating an inflammatory condition in a mammal exhibiting a biochemical signature characterized by a down-regulation of UCHL1 expression.

### SUMMARY OF THE INVENTION:

The present invention is defined by the claims. In particular, the present invention relates to:
i) an *in vitro* method of determining whether a subject suffers from idiopathic steroid sensitive nephrotic syndrome (INS) comprising i) determining the concentration of plasma anti- UCHL1 IgG in a blood sample obtained from the subject ii) comparing the concentration determined at step i) with a predetermined reference value and iii) concluding that the subject suffer from idiopathic steroid sensitive nephrotic syndrome when the concentration determined at step i) is higher than the predetermined reference value.
ii) an *in vitro* method of predicting the risk of relapse in a subject suffering from INS i) comprising determining the concentration of plasma anti-UCHLl IgG in a blood sample obtained from the subject ii) comparing the concentration determined at step i) with a predetermined reference value and iii) concluding that the subject is at risk of relapse when the concentration determined at step i) is higher than the predetermined reference value
iii) an *in vitro* method of determining whether the subject achieves a response with an immunosuppressive treatment comprising i) determining the concentration of plasma anti-UCHL1 IgG in a blood sample obtained from the subject before the treatment ii) determining the concentration of plasma anti-UCHLl IgG in a sample obtained from the subject after the treatment, iii) comparing the concentration determined at step i) with the concentration determined at step ii) and iv) concluding that the subject achieves a response when the concentration determined at step ii) lower than the concentration determined at step i), and
iv) at least one immunosuppressive agent for use in the treatment of idiopathic steroid sensitive nephrotic syndrome (INS) in a subject in need thereof comprising the steps of: i) diagnosing the subject by the method of claim 1 and ii) administering to the said subject a therapeutically effective amount of at least one immunosuppressive agent wherein the immunosuppressive agent is selected from the group consisting of 6-mercaptopurine ("6-MP"), cyclophosphamide, mycophenolate, prednisolone, sirolimus, dexamethasone, rapamycin, FK506, mizoribine, azathioprine, tacrolimus, calcineurin inhibitors, corticosteroids, and B cell depleting agents.

### DETAILED DESCRIPTION:

While numerous clinical facts including the efficacy of drugs targeting the B cells and the association of memory B cells with relapses, the involvement of immunoglobulin G (IgG) might be reconsidered in with idiopathic nephrotic syndrome (INS). The aim of the inventors was to identify antibodies directed against podocyte molecular targets in subjects with steroid sensitive INS. Detachment of podocytes was observed with plasma concentration over 13%, either from control or subjects, and was prevented by anti-IgG neutralization. A significant detachment of podocyte was observed with 47% of pooled fractions containing IgG from INS proteinuria whereas the same fractions from 25 controls and 91% from those of INS subjects in remission had no effect on podocyte adhesion. A list of podocyte proteins was established after immunoprecipitation of podocyte lysates with IgG from the plasma fractions of interest and proteomic analysis. Five podocyte targets were selected on statistical criterias and relevance in podocyte biology. Specific antibodies of those proteins were tested and only anti-Ubiquitin Carboxyl-Terminal Hydrolase L1 (UCHL1) IgG led to podocyte detachment. Incubation of either Anti-UCHL1 IgG or plasma fractions of interest with recombinant UCHL1 prevented podocyte detachment. The concentration of plasma anti-UCHL1 IgG was significantly increased in INS proteinuric subjects compared to control children and INS remission subjects. Proteinuria was significantly correlated with plasma concentration of anti-UCHL1 IgG in selected subjects (42% of the series) with a high plasma concentration of anti-UCHL1 IgG during the proteinuria phase.

The first object disclosed herein relates to a method of determining whether a subject suffers from idiopathic steroid sensitive nephrotic syndrome (INS) comprising i) determining the concentration of anti-UCHL1 IgG in a sample obtained from the subject ii) comparing the concentration determined at step i) with a predetermined reference value and iii) concluding that the subject suffer from idiopathic steroid sensitive nephrotic syndrome when the concentration determined at step i) is higher than the predetermined reference value.

As used herein the term "idiopathic steroid sensitive nephrotic syndrome" or "INS" has its general meaning in the art and refers to a kidney disease defined by selective proteinuria, hypoalbuminaemia and, on renal biopsy, minimal changes without immunoglobulin deposits. The annual incidence in children is 1 to 1.5 cases per 50 000 with a prevalence of 1 cases per 6 250. Conversely, the annual incidence in adults is only one case per 330 000. There is an incidence peak between the ages of 2 and 6 years. INS is marked by sudden onset, oedema being the major symptom. According to the present disclosure, the subject is typically a child between 2 and 6 years old.

In particular, the method of diagnosing described herein is applied to a subject who presents symptoms of INS without having undergone the routine screening to rule out all possible causes for INS. The methods described herein can be part of the routine set of tests performed on a subject who presents symptoms of INS such as proteinuria for diagnostic purposes. Such subjects have not been biopsied for the confirmatory diagnosis of INS. Similarly, the method can be part of the routine set of serological immunological tests performed for a subject who already is known to have INS by biopsy or by serological testing, is being treated for INS.

As used herein, the term "sample" includes any liquid or fluid sample, liable to contain anti-UCHL1 IgG. In particular, the sample is selected from the group consisting of ascites; urine; saliva; sweat; milk; synovial fluid; peritoneal fluid; amniotic fluid; percerebrospinal fluid; lymph fluid; lung embolism; cerebrospinal fluid; and pericardial fluid;
In particular, the sample is a urine sample.

In particular, the sample is a blood sample. As used herein the term "blood sample" means any blood sample derived from the subject. Collections of blood samples can be performed by methods well known to those skilled in the art. For example, the subject' s blood can be drawn by trained medical personnel directly into anti-coagulants such as citrate and EDTA. The whole blood can be separated into the plasma portion, the cells, and platelets portion by refrigerated centrifugation at 3500 X G for 2 minutes. After centrifugation, the supernatant is the plasma.

As used herein the term "UCHL1" has its general meaning in the art and refers to the ubiquitin C-terminal hydrolase L1 encoded by the UCHL1 gene (gene ID : 7345). The term is also known as NDGOA; PARK5; PGP95; PGP9.5; Uch-L1; HEL-117; PGP 9.5; and HEL-S-53. A human exemplary amino acid sequence is represented by the NCBI reference sequence NP_004172.2.

As use herein, the term "IgG" means a class of immunoglobulins including the most common antibodies circulating in the blood that facilitate the phagocytic destruction of microorganisms foreign to the body, that bind to and activate complement, and that are the only immunoglobulins to cross over the placenta from mother to fetus. Immunoglobulin G (IgG) is an antibody isotype. It is a protein complex composed of four peptide chains-two identical heavy chains and two identical light chains arranged in a Y-shape typical of antibody monomers. Each IgG has two antigen binding sites. Representing approximately 75% of serum immunoglobulins in humans, IgG is the most abundant antibody isotype found in the circulation. IgG molecules are synthesized and secreted by plasma B cells. There are four IgG subclasses (IgG 1, 2, 3, and 4) in humans, named in order of their abundance in serum (IgG1 being the most abundant).

As use herein the term "anti-UCHL1 IgG" refers to the immunoglobulin (i.e. antibodies) which are produced by the immune system of the subject and that are directed against subject's UCHL1 own protein.

A further object disclosed herein relates to a method of predicting the risk of relapse in a subject suffering from INS i) comprising determining the concentration of plasma anti-UCHL1 IgG in a sample obtained from the subject ii) comparing the concentration determined at step i) with a predetermined reference value and iii) concluding that the subject is at risk of relapse when the concentration determined at step i) is higher than the predetermined reference value.

As used herein, the term "risk" in the context of the present disclosure, relates to the probability that an event will occur over a specific time period, as in the conversion to relapse, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(1-p) where p is the probability of event and (1- p) is the probability of no event) to no- conversion. "Risk evaluation," or "evaluation of risk" in the context of the present disclosure encompasses making a prediction of the probability, odds, or likelihood that an event or disease state may occur, the rate of occurrence of the event or conversion from one disease state to another, i.e., from a normal condition to relapse or to one at risk of developing relapse. Risk evaluation can also comprise prediction of future clinical parameters, traditional laboratory risk factor values, or other indices of relapse, either in absolute or relative terms in reference to a previously measured population. The methods disclosed herein may be used to make continuous or categorical measurements of the risk of conversion to relapse, thus diagnosing and defining the risk spectrum of a category of subjects defined as being at risk of having relapse. In the categorical scenario, the method disclosed herein can be used to discriminate between normal and other subject cohorts at higher risk of having relapse. In particular, the method disclosed herein may be used so as to discriminate those at risk of having relapse from normal, or those having relapse disease from normal.

As used herein, the term "relapse" refers to the return of signs and symptoms of a disease after a subject has enjoyed a remission after a treatment. Thus, if initially the target disease is alleviated or healed, or progression of the disease was halted or slowed down, and subsequently the disease or one or more characteristics of the disease resume (e;g. proteinuria), the subject is referred to as being "relapsed." Typically, the treatment is an immunosuppressive treatment.

Typically, the predetermined reference value is a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the expression level of the selected peptide in a group of reference, one can use algorithmic analysis for the statistic treatment of the expression levels determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In particular, the predetermined reference value is the concentration of plasma anti-UCHL1 IgG determined in a population of healthy individuals. Typically, it is concluded that the patient suffers from INS or is at risk of relapse when the concentration of plasma anti-UCHL1 IgG is at least 0.5, 1, 1.5, 2, 2.5, 3, 3.5, 4, 5, 6, 7, 8, 9, 10, 15, 20, 30, 40, 50, 100 fold higher than the concentration determined in a population of healthy individuals.

A further object disclosed herein relates to a method of determining whether the subject achieves a response with an immunosuppressive treatment comprising i) determining the concentration of plasma anti-UCHLl IgG in a sample obtained from the subject before the treatment ii) determining the concentration of plasma anti-UCHLl IgG in a sample obtained from the subject after the treatment, iii) comparing the concentration determined at step i) with the concentration determined at step ii) and iv) concluding that the subject achieves a response when the concentration determined at step ii) lower than the concentration determined at step i).

The method is thus particularly suitable for discriminating responder from non-responder. As used herein the term "responder" in the context of the present disclosure refers to a subject that will achieve a response, i.e. a subject who is under remission and more particularly a subject who does not suffers from proteinuria. A non-responder subject includes subjects for whom the disease does not show reduction or improvement after the immunosuprresive treatment (e.g. the proteinuria remains stable or increases.

As used herein, the term "immunosuppressive treatment" refers to any substance capable of producing an immunosuppressive effect, e.g., the prevention or diminution of the immune response and in particular the prevention or diminution of the production of IgG. Immunosuppressive drugs include, without limitation thiopurine drugs such as azathioprine (AZA) and metabolites thereof; nucleoside triphosphate inhibitors such as mycophenolic acid (Cellcept) and its derivative (Myfortic); derivatives thereof; prodrugs thereof; and combinations thereof. Other examples include but are not limited to 6-mercaptopurine ("6-MP"), cyclophosphamide, mycophenolate, prednisolone, sirolimus, dexamethasone, rapamycin, FK506, mizoribine, azothioprine and tacrolimus.

In particular the immunosuppressive drug is a calcineurin inhibitor. As used herein, the term "calcineurin inhibitor" has its general meaning in the art and refers to substances which block calcineurin (i.e. calcium/calmodulin-regulated protein phosphatase involved in intracellular signalling) dephosphorylation of appropriate substrates, by targeting calcineurin phosphatase (PP2B, PP3), a cellular enzyme that is involved in gene regulation. A calcineurin inhibitor disclosed herein is typically an immunophilin-binding compound having calcineurin inhibitory activity. Immunophilin-binding calcineurin inhibitors are compounds forming calcineurin inhibiting complexes with immunophilins, e.g. cyclophilin and macrophilin. Examples of cyclophilin-binding calcineurin inhibitors are cyclosporines or cyclosporine derivatives (hereinafter cyclosporines) and examples of macrophilin-binding calcineurin inhibitors are ascomycin (FR 520) and ascomycin derivatives (hereinafter ascomycins). A wide range of ascomycin derivatives are known, which are either naturally occurring among fungal species or are obtainable by manipulation of fermentation procedures or by chemical derivatization. Ascomycin-type macrolides include ascomycin, tacrolimus (FK506), sirolimus and pimecrolimus. Cyclosporine, originally extracted from the soil fungus Potypaciadium infilatum, has a cyclic 11-amino acid structure and includes e.g. Cyclosporines A through I, such as Cyclosporine A, B, C, D and G. Voclosporin is a next-generation calcineurin inhibitor that is a more potent and less toxic semi-synthetic derivative of cyclosporine A. In particular, the calcineurin inhibitor disclosed herein is the trans-version of voclosporin, trans-ISA247 (Cas number 368455-04-3) which is described in, for example, US Patent Publication No.: 2006/0217309. Further compositions of voclosporin are described, for example, in U.S. Pat. No. 7,060,672. Tacrolimus (FK506) is another calcineurin inhibitor which is also a fungal product, but has a macrolide lactone structure. Sirolimus (rapamycin) is a microbial product isolated from the actinomycete Streptomyces hygroscopicus. Sirolimus binds to an immunophilin (FK-binding protein 12, FKBP12) forming a complex, which inhibits the mammalian target of rapamycin (mTOR) pathway through directly binding the mTOR Complex1 (mTORC1). Pimecrolimus is also a calcineurin inhibitor. Calcineurin inhibitors such as cyclosporine A, voclosporin, ascomycin, tacrolimus, pimecrolimus, an analog thereof, or a pharmaceutically acceptable salt thereof, can be utilized in a mixed micellar composition of the present disclosure.

In particular, the immunosuppressive drug is a corticosteroid. As used, the term "corticosteroids" has its general meaning in the art and refers to class of active ingredients having a hydrogenated cyclopentoperhydrophenanthrene ring system endowed with an antiinflammatory activity. Corticosteroid drugs typically include cortisone, cortisol, hydrocortisone (11β,17-dihydroxy, 21-(phosphonooxy)-pregn-4-ene, 3,20-dione disodium), dihydroxycortisone, dexamethasone (21-(acetyloxy)-9-fluoro-1β,17-dihydroxy-16α-m-ethylpregna-1,4-diene-3,20-dione), and highly derivatized steroid drugs such as beconase (beclomethasone dipropionate, which is 9-chloro-11-β,17,21,trihydroxy-160-methylpregna-1,4 diene-3,20-dione 17,21-dipropionate). Other examples of corticosteroids include flunisolide, prednisone, prednisolone, methylprednisolone, triamcinolone, deflazacort and betamethasone. corticosteroids, for example, cortisone, hydrocortisone, methylprednisolone, prednisone, prednisolone, betamethesone, beclomethasone dipropionate, budesonide, dexamethasone sodium phosphate, flunisolide, fluticasone propionate, triamcinolone acetonide, betamethasone, fluocinolone, fluocinonide, betamethasone dipropionate, betamethasone valerate, desonide, desoximetasone, fluocinolone, triamcinolone, triamcinolone acetonide, clobetasol propionate, and dexamethasone.

In particular, the immunosuppressive drug is a B cell depleting agent. As used herein, the term "B cell depleting agent" refers to any agent that is capable of triggering lymphodepletion of B cells. In particular, the B cell depleting agent is an antibody having specificity for CD20. Examples of antibodies having specificity for CD20 include: "C2B8" which is now called "Rituximab" ("RITUXAN®") (U.S. Pat. No. 5,736,137), a chimaeric pan-B antibody targeting CD20; the yttrium-[90]-labeled 2B8 murine antibody designated "Y2B8" or "Ibritumomab Tiuxetan" ZEVALIN® (U.S. Pat. No. 5,736,137), a murine IgG1 kappa mAb covalently linked to MX-DTPA for chelating to yttrium-[90]; murine IgG2a "BI," also called "Tositumomab," optionally labeled with radioactive 1311 to generate the "1311-B1" antibody (iodine 131 tositumomab, BEXXAR™) (U.S. Pat. No. 5,595,721); murine monoclonal antibody "1F5" (Press et al. Blood 69 (2):584-591 (1987) and variants thereof including "framework patched" or humanized 1F5 (WO03/002607, Leung, S.; ATCC deposit HB-96450); murine 2H7 and chimeric 2H7 antibody (U.S. Pat. No. 5,677,180); humanized 2H7, also known as ocrelizumab (PRO-70769); Ofatumumab (Arzerra), a fully human IgG1 against a novel epitope on CD20 huMax-CD20 (Genmab, Denmark; WO2004/035607 (U.S. Ser. No. 10/687,799)); AME-133 (ocaratuzumab; Applied Molecular Evolution), a a fully-humanized and optimized IgG1 mAb against CD20; A20 antibody or variants thereof such as chimeric or humanized A20 antibody (cA20, hA20, respectively) (U.S. Ser. No. 10/366,709, Immunomedics); and monoclonal antibodies L27, G28-2, 93-1B3, B-CI or NU-B2 available from the International Leukocyte Typing Workshop (Valentine et al, In: Leukocyte Typing III (McMichael, Ed., p. 440, Oxford University Press (1987)). Further, suitable antibodies include e.g. antibody GA101 (obinutuzumab), a third generation humanized anti-CD20-antibody of Biogen Idec/Genentech/Roche. Moreover, BLX-301 of Biolex Therapeutics, a humanized anti CD20 with optimized glycosylation or Veltuzumab (hA20), a 2nd-generation humanized antibody specific for CD20 of Immunomedics or DXL625, derivatives of veltuzumab, such as the bispecific hexavalent antibodies of IBC Pharmaceuticals (Immunomedics) which are comprised of a divalent anti-CD20 IgG of veltuzumab and a pair of stabilized dimers of Fab derived from milatuzumab, an anti-CD20 mAb enhanced with InNexus' Dynamic Cross Linking technology, of Inexus Biotechnology both are humanized anti-CD20 antibodies are suitable. Further suitable antibodies are BM-ca (a humanized antibody specific for CD20 (Int J. Oncol. 2011 February; 38(2):335-44)), C2H7 (a chimeric antibody specific for CD20 (Mol Immunol. 2008 May; 45(10):2861-8)), PRO131921 (a third generation antibody specific for CD20 developed by Genentech), Reditux (a biosimilar version of rituximab developed by Dr Reddy's), PBO-326 (a biosimilar version of rituximab developed by Probiomed), a biosimilar version of rituximab developed by Zenotech, TL-011 (a biosimilar version of rituximab developed by Teva), CMAB304 (a biosimilar version of rituximab developed by Shanghai CP Guojian), GP-2013 (a biosimilar version of rituximab developed by Sandoz (Novartis)), SAIT-101 (a biosimilar version of rituximab developed by Samsung BioLogics), a biosimilar version of rituximab developed by Intas Biopharmaceuticals, CT-P10), a biosimilar version of rituximab developed by Celltrion), a biosimilar version of rituximab developed by Biocad, Ublituximab (LFB-R603, a transgenically produced mAb targeting CD20 developed by GTC Biotherapeutics (LFB Biotechnologies)), PF-05280586 (presumed to be a biosimilar version of rituximab developed by Pfizer), Lymphomun (Bi-20, a trifunctional anti-CD20 and anti-CD3 antibody, developed by Trion Pharma), a biosimilar version of rituximab developed by Natco Pharma, a biosimilar version of rituximab developed by iBio, a biosimilar version of rituximab developed by Gedeon Richter/Stada, a biosimilar version of rituximab developed by Curaxys, a biosimilar version of rituximab developed by Coherus Biosciences/Daiichi Sankyo, a biosimilar version of rituximab developed by BioXpress, BT-D004 (a biosimilar version of rituximab developed by Protheon), AP-052 (a biosimilar version of rituximab developed by Aprogen), a biosimilar version of ofatumumab developed by BioXpress, MG-1106 (a biosimilar version of rituximab developed by Green Cross), IBI-301 (a humanized monoclonal antibody against CD20 developed by Innovent Biologics), BVX-20 (a humanized mAb against the CD20 developed by Vaccinex), 20-C2-2b (a bispecific mAb-IFNalpha that targets CD20 and human leukocyte antigen-DR (HLA-DR) developed by Immunomedics), MEDI-552 (developed by MedImmune/AstraZeneca), the anti-CD20/streptavidin conjugates developed by NeoRx (now Poniard Pharmaceuticals), the 2nd generation anti-CD20 human antibodies developed by Favrille (now MMRGlobal), TRU-015, an antibody specific for CD20 fragment developed by Trubion/Emergent BioSolutions, as well as other precloinical approaches by various companies and entities. All antibodies disclosed in therein may be used within the present disclosure.

A decrease in the concentration after the treatment compared to the concentration before the treatment of at least 5%, at least 10%, at least 20%, at least 30%, at least 40%, at least 50%, at least 60%, at least 70%, at least 80%, at least 90%, 100% typically indicated that the subject achieves a response. More preferably a concentration which returns to the basal concentration (i.e. the concentration determined in a population of healthy individuals) indicates that the subject has achieved a response.

The detection and quantification of anti-UCHLl IgG in the sample can be detected by any method known in the art. Typically the detection and quantification is performed by Enzyme-linked immunosorbent assay, also called ELISA, enzyme immunoassay or EIA, is a biochemical technique used mainly in immunology to detect the presence of an antibody or an antigen in a sample. A known amount of antigen (UCHL1) is immobilized on a solid support (usually a polystyrene micro titer plate) either non-specifically (via adsorption to the surface) or specifically (via capture by another antibody specific to the same antigen, in a "sandwich" ELISA). Then the sample, suspected of containing anti-UCHLl IgG, is washed over the surface so that the auto-antibodies can bind to the immobilized antigen. The surface is washed to remove any unbound protein and a detection antibody is applied to the surface. The detection antibody should be an anti-human IgG antibody. The detection antibody can be covalently linked to an enzyme, or can itself be detected by a secondary antibody which is linked to an enzyme through bio-conjugation. Enzymes which can be used to detectably label the antibodies disclosed herein include, but are not limited to, malate dehydrogenase, staphylococcal nuclease, delta- V- steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose- VI-phosphate dehydrogenase, glucoamylase and acetylcholinesterase. Between each step the plate is typically washed with a mild detergent solution to remove any proteins or antibodies that are not specifically bound. After the final wash step the plate is developed by adding an enzymatic substrate to produce a visible signal, which indicates the quantity of antigen in the sample. In particular, a competitive ELISA is used. Purified anti-UCHLl antibodies that are not derived from the subject are coated on the solid phase of multi- wells. Serum sample recombined UCHL1, (the antigen) or fragments thereof and horseradish peroxidase labeled with anti-UCHL1 antibodies (conjugated) are added to coated wells, and form competitive combination. After incubation, if the auto-antibody level against UCHL1 content is high in the sample, a complex of UCHL1-auto-antibodies-anti-UCHL1 labeled with HRP will form. Wash wells will remove the complex, and incubate with TMB (3, 3', 5, 5'- tetramethylbenzidene) color development substrate for localization of horseradish peroxidase- conjugated antibodies in the wells. Subsequently there will be no color change or little color change. If there are no auto-anti-UCHLl IgG in the serum sample, there will be much color change. Such a competitive ELSA test is specific, sensitive, reproducible and easy to operate. In particular, the detection antibody is label with a fluorescent compound. When the fluorescently labeled antibody is exposed to light of the proper wavelength, its presence can then be detected due to fluorescence. Among the most commonly used fluorescent labeling compounds are CY dyes, fluorescein isothiocyanate, rhodamine, phycoerytherin, phycocyanin, allophycocyanin, o-phthaldehyde and fluorescamine. In particular the detection antibody can also be detectably labeled using fluorescence emitting metals such as 152Eu, or others of the lanthanide series. These metals can be attached to the antibody using such metal chelating groups as diethylenetriaminepentaacetic acid (DTPA) or ethylenediaminetetraacetic acid (EDTA). In particular, the detection antibody is detectably labeled by coupling it to a chemiluminescent compound. The presence of the chemiluminescent-antibody is then determined by detecting the presence of luminescence that arises during the course of a chemical reaction. Examples of particularly useful chemiluminescent labeling compounds are luminol, luciferin, isoluminol, theromatic acridinium ester, imidazole, acridinium salt and oxalate ester. There are other different forms of ELISA, which are well known to those skilled in the art. In particular, the immunoassays comprise beads coated with native or recombinant UCHL1 protein as described. Commonly used are polystyrene beads that are dyed to establish a unique identity. Detection is performed by flow cytometry. Autoantibody detection using multiplex technologies. Other types of bead- based immunoassays are well known in the art, e. g. laser bead immunoassays and related magnetic bead assays (Fritzler, Marvin J; Fritzler, Mark L, Expert Opinion on Medical Diagnostics, 2009, pp. 3: 81-89).

A further object disclosed herein relates to a kit or device for identifying the presence or the concentration of anti-UCHLl IgG in a sample from a subject comprising: at least a UCHL1 protein or fragments thereof; and at least one solid support wherein the UCHL1 protein or fragments thereof is deposited on the support. In particular, the UCHL1 protein or fragments thereof that is deposited on the solid support is immobilized on the support. In particular, the solid support is in the format of a dipstick, a test strip, a latex bead, a microsphere or a multi-well plate. In particular, the devices or kits described herein can further comprise a second labeled UCHL1 protein or a fragment thereof which produces a detectable signal; a detection antibody, wherein the detection antibody is specific for the anti-UCHLl IgG in the sample of the subject and the detection antibody produces a detectable signal; or a nephelometer cuvette. In particular, the device performs an immunoassay wherein an antibody-protein complex is formed, such as a serological immunoassay or a nephelometric immunoassay. In particular, provided herein are kits that comprise devices described herein and a detection antibody, wherein the detection antibody is specific for the anti-UCHLl IgG in the sample of the subject and produces a detectable signal. In particular, the kit can include a second labeled UCHL1 protein or a fragment thereof which produces a detectable signal. In particular, the kit includes a nephelometer cuvette. Any solid support can be used, including but not limited to, nitrocellulose membrane, nylon membrane, solid organic polymers, such as polystyrene, or laminated dipsticks such as described in U.S. patent 5,550,375. The use of "dip sticks" or test strips and other solid supports have been described in the art in the context of an immunoassay for a number of antigens. Three U.S. patents (U.S. Pat. No. 4,444,880, issued to H. Tom; U.S. Pat. No. 4,305,924, issued to R. N. Piasio; and U.S. Pat. No. 4,135,884, issued to J. T. Shen) describe the use of "dip stick" technology to detect soluble antigens via immunochemical assays. The apparatuses and methods of these three patents broadly describe a first component fixed to a solid surface on a "dip stick" which is exposed to a solution containing a soluble antigen that binds to the component fixed upon the "dip stick," prior to detection of the component-antigen complex upon the stick. The "dip stick" technology can be easily adapted for the present disclosure by one skilled in the art. In the present disclosure, the antigen UCHL1 is deposited on the support and the auto-antibody is to be detected. Examples of kits include but are not limited to ELISA assay kits, and kits comprising test strips and dipsticks. In an ELISA kit, an excess amount of UCHL1 antigen, in, is immobilized on a solid support. A sample containing an unknown amount of anti-UCHLl IgG is added to the immobilized UCHL1, resulting in the formation of a complex consisting of the protein and the antibody. The complex is detected by a labeled second antibody that is also specific for the auto-antibody. In particular of the kits described herein, the kit comprises a test strip or a dipstick. In a typical colloidal gold labeling technique, the unique red color of the accumulated gold label, when observed by lateral or transverse flow along a membrane on which an antigen is captured by an immobilized antibody, or by observation of the red color intensity in solution, provides an extremely sensitive method for detecting sub nanogram quantities of proteins in solution. A colloidal gold conjugate consists of a suspension of gold particles coated with a selected protein or macromolecule (such as an antibody or antibody- based moiety). The gold particles may be manufactured to any chosen size from 1-250nm. This gold probe detection system, when incubated with a specific target, such as in a tissue section, will reveal the target through the visibility of the gold particles themselves. For detection by eye, gold particles will also reveal immobilized antigen on a solid phase such as a blotting membrane through the accumulated red color of the gold sol. Silver enhancement of this gold precipitate also gives further sensitivity of detection. Suppliers of colloidal gold reagents for labeling are available from SPI-MARK™. Polystyrene latex Bead size 200 nm colored latex bead coated with antibody SIGMA ALDRICH®, Molecular Probes, Bangs Laboratory Inc., and AGILENT® Technologies. In particular of the kits described herein, at least one of the labeled antibodies comprises an enzyme-labeled antibody. The anti-UCHLl that is bound and captured by the immobilized UCHL1 on the solid support (e. g. microtiter plate wells) is identified by adding a chromogenic substrate for the enzyme conjugated to the anti- antibody, e. g. anti-human IgG, and color production detected by an optical device such as an ELISA plate reader. In particular, the kits described herein further comprise standards of known amounts of the UCHL1 or fragments thereof. In particular, the kits described herein further comprise reference values of the levels of anti-UCHLl antibodies. The reference values are average levels of anti-UCHLl antibodies in samples from a population of healthy individuals. Reference values can be provided as numerical values, or as standards of known amounts or titer of anti-UCHLl antibodies presented in pg/ml-µg/ml. In particular, the kits described herein further comprise at least one sample collection container for sample collection. Collection devices and container include but are not limited to syringes, lancets, BD VACUTAINER® blood collection tubes. In particular, the kits described herein further comprise instructions for using the kit and interpretation of results.

A further object disclosed herein relates to systems (and computer readable media for causing computer systems) to perform a method for diagnosing INS in a subject, assessing a subject's risk of developing INS, or monitoring treatment efficacy of a subject with INS. In particular, the system comprise a measuring module measuring auto-antibody information comprising a detectable signal from an immunoassay indicating the presence or concentration of anti-UCHLl IgG from a sample obtained from a subject; a storage module configured to store data output from the measuring module; a comparison module adapted to compare the data stored on the storage module with reference and/or control data, and to provide a retrieved content, and an output module for displaying the retrieved content for the user, wherein the retrieved content the presence of detectable amount of antibodies reactive against UCHL1 indicates that the subject has INS or has a relapse of INS. In particular, the control data comprises previous data from the same subject wherein the previous data had indicated detectable amounts of auto-antibodies. Embodiments of the disclosure can be described through functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions. The computer readable storage media can be any available tangible media that can be accessed by a computer. Computer readable storage media includes volatile and nonvolatile, removable and non-removable tangible media implemented in any method or technology for storage of information such as computer readable instructions, data structures, program modules or other data. The computer-readable media may be transportable such that the instructions stored thereon can be loaded onto any computer resource to implement the aspects discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects disclosed herein. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

In some aspects, the removal of the anti-UCHL1 IgG is expected to be of therapeutic value. Accordingly a further object disclosed herein relates to a method of treating a subject suffering from idiopathic steroid sensitive nephrotic syndrome (INS) by removing anti-UCHLl IgG from body fluid from the subject comprising the steps of a) providing the extracellular body fluid (e.g. blood) which has been obtained from a subject, b) contacting the extracellular body fluid with a biocompatible solid support capable of capturing the anti-UCHLl IgG, and c) reinfusing the extracellular body fluid from step into the subject.

In extracorporeal immunoadsorption, circulating antibodies are extracorporeally removed using an immunoadsorbent column specific for the endogenous antibody. Blood from the patient is withdrawn either continuously or discontinuously, separated into its cellular components and plasma, and the plasma is perfused through the immunoadsorbent material in order to remove the antibody. The treated plasma and cellular components of the blood are then reinfused into the patient, either separately or simultaneously.

Specific removal of circulating antibodies by extracorporeal immunoadsorption employing an immobilized antigen has been described by various investigators. See generally Kohler et al., (201 1) J Clin Apher. (6):347-55; Muller et al., (2012) Dermatology.;224(3):224-7; Koziolek et al., (2012) J Neuroinflammation. 9(1):80; Bontadi et al., (2012) J Clin Apher. doi: 10.1002/jca.21229; Westermann et al., (2012) J Dermatol. 39(2): 168-71. Moreover this approach has been successfully commercialized as a viable system to specifically remove circulating antibodies, as exemplified by immunoadsorption columns sold under the trademarks Prosorba®, Immunosorba®, sold by Fresenius, St. Wendel, Germany, and Selesorb® sold by Kaneka, Wiesbaden, Germany.

In particular, an amount of Protein A or Protein G is immobilized in the solid support. Protein A or Protein G (for example, obtained from Miltenyi Biotec, Germany) are components of the cell wall of the bacterium Staphylococcus and have the capacity to bind non-selective immunoglobulins of the IgG class because of their high affinity to the Fc portion of the IgG antibodies (Class 1, 2 and 4).

In particular, an amount of a recombinant UCHL1 polypeptide is immobilized in the solid support. In particular, the immunoadsorption is more specific so that only IgG specific to UCHL1 are captured on the solid support; the other IgG are eluted in the extracellular body fluid.

In general, in any of these immunoadsorbent methods and compositions, the body fluids are obtained, handled and re-infused under aseptic conditions using methods and systems that are well known to a person skilled in the art. For example, blood is withdrawn via a needle that is introduced into, for example, a peripheral vein connected via a suitable tube to the container containing the biocompatible solid support and re-infused into the patient via an inlet tube connected to a needle inserted into another vein. In situations where large volumes are to be withdrawn from the subject, blood may be withdrawn, for instance, from the vena subclavia. Optionally, an anticoagulation substance such as sodium citrate, heparin, or dextran can be added to the blood when withdrawn from the body to prevent coagulation of the blood. Dextran reduces the viscosity of the blood and, in combination with addition of saline, ensures an increased distance between the blood cells and the blood platelets. Such anticoagulants may be added in quantities sufficient for non-coagulation of the blood. Before reinfusion of the treated blood into the subject the anticoagulation effect of e.g. heparin, may be reduced with the appropriate amount of heparinase, protamine and/or vitamin K etc.

Suitable columns and perfusion systems for extracorporeal adsorption are commercially available, for example from Fresenius, St. Wendel, Germany. Contact temperatures in the range of 35°C to about 40°C are typically used. The contact time will typically be in the range of about 1 to about 6 hours. The unbound portion of the blood or plasma is then collected for reintroduction into the patient or it can be reintroduced directly on a continuous basis. The subject's anti-UCHLl IgG titer may be monitored by immunoassay before and /or after the procedure to monitor the efficiency of the procedure.

To reduce the risk of embolism, precautions can be taken to avoid adsorption medium particles entering the patient upon reinfusion. Accordingly a particle capture device is typically employed downstream of the adsorption medium container to remove any residual particles from the remainder of the body fluid before it is returned to the patient. The particle capture device may be a filter or mesh having openings of a size that retain any particulate material of the adsorption medium while letting the non-adsorbed entities of the body fluid pass through. The extracorporeal blood perfusion may be performed continuously, or alternatively, discrete volumes of blood may be removed from the patient, treated as described above, and the treated plasma and cellular components of the blood returned to the patient after the treatment is complete.

A wide variety of materials will be suitable as biocompatible solid supports, for use in any of these immunoadsorbent methods and compositions, and ideally, the support matrix will be mechanically strong, sufficiently hydrophilic to avoid non-specific binding of proteins, stable and compatible with to blood and other aqueous solutions. Suitable biocompatible matrix materials include, for example, synthetic and natural polymers, polysaccharides, polyamides, glass beads, particulate silica, porous glass, silica, resins, synthetic matrixes including acrylamide derivatives, methacrylamide derivatives or polystyrene derivatives, etc, in various forms including beads, fibrous form, sheets or hollow fibers. Exemplary polymers include natural and synthetic polysaccharides and other carbohydrate based polymers, including agar, alginate, carrageenan, guar gum, gum arabic, gum ghatti, gum tragacanth, karaya gum, locust bean gum, xanthan gum, agaroses, celluloses, pectins, mucins, dextrans, starches, heparins, chitosans, hydroxy starches, hydroxypropyl starches, carboxymethyl starches, hydroxyethyl celluloses, hydroxypropyl celluloses, and carboxymethyl celluloses. Synthetic organic polymers and monomers resulting in polymers, including acrylic polymers, polyamides, polyimides, polyesters, polyethers, polymeric vinyl compounds, polyalkenes, and substituted derivatives thereof, as well as copolymers comprising more than one such polymer functionality, and substituted derivatives thereof; and mixtures thereof.

In any of these extracorporeal methods and compositions, the UCHL1 polypeptides are typically, covalently coupled to the biocompatible solid support, and standard methods for coupling proteins such as the UCHL1 polypeptides are well known to those of skill in the art (see. e.g. Affinity Chromatography, Principles and Methods (Pharmacla-LKB), Dean,P.G., et al., eds., 1985, Affinity Chromatography: A practical approach, IRL Press, Oxford, and Scouten, W.H., 1981, Affinity Chromatography, Wiley Intersclence, New York), "Immobilized Affinity Ligand Techniques" by Hermanson et al., Academic Press, Inc., San Diego, 1992). The biocompatible solid support may be derivatized (activated) to form a reactive substance that can react with one or more functional chemical groups within the UCHL1 polypeptide, thereby forming a chemical covalent bond to couple the UCHL1 polypeptide to the biocompatible solid support. Thus, materials comprising hydroxyl, amino, amide, carboxyl or thiol groups may be activated or derivatized using various activating chemicals, e.g., chemicals such as cyanogen bromide, divinyl sulfone, epichlorohydrin, bisepoxyranes, dibromopropanol, glutaric dialdehyde, carbodiimides, anhydrides, hydrazines, periodates, benzoquinones, triazines, tosylates, tresylates, and/or diazonium ions, etc. Specific exemplary activated biocompatible solid supports for use in any of these methods and compositions include for example CNBr-Sepharose, celluloses, such as CNBr- activated Sepharose 4B (Amersham), or Epoxy-activated agarose (Sigma). Biocompatible spacers (like for example NHS-activated Sepharose 4 Fast Flow) or without (like for example CNBr- activated Sepharose 4B) may be employed and are commercially available, and methods for coupling such materials to UCHL1 polypeptides are well known in the art, and can be optimized by routine experimentation based on the manufacturer's directions.

The present disclosure will be further illustrated by the following figures and examples. However, these examples and figures should not be interpreted in any way as limiting the scope of the present disclosure.

### FIGURES:

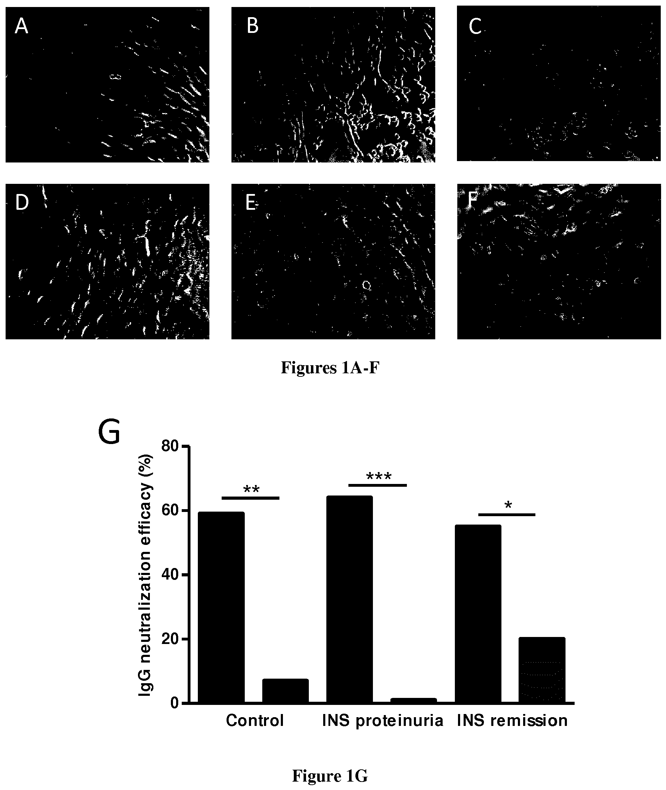

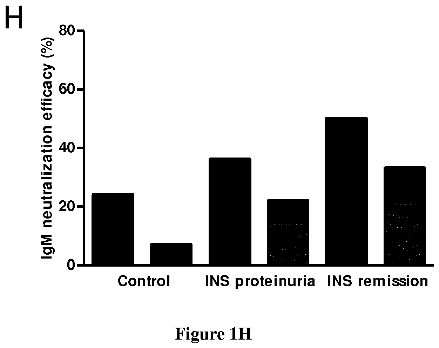

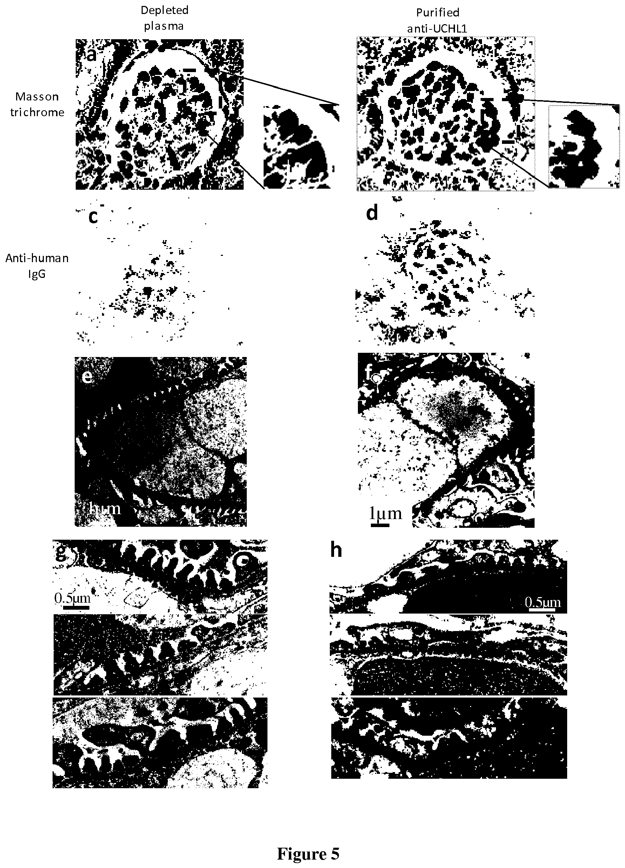

**Figure 1****. Effect of plasma exposure on morphology of podocytes and neutralization with monoclonal antibodies.** (A) Adherent cultured podocytes, (B) retracted podocytes incubated for 2 h with 9% plasma and (C) detached podocytes incubated for 2h with 13% plasma, magnification x10. Adherent podocytes incubated for 2 h with (D) 20% PBS and (E)20% FCS, magnification x10. (F) Neutralization of plasma able to detach podocytes with monoclonal anti-IgG for 30 min at 37°C before adding 13 % of this treated plasma on podocytes avoid cell detachment, magnification x10. (G) Neutralization efficacy of 13% plasma exposure for 2h on podocytes after plasma neutralization with monoclonal anti-IgG or isotype control for 30 min at 37°C. (H) Neutralization efficacy of 13% plasma exposure for 2 h on podocytes after plasma neutralization with monoclonal anti-IgM or isotype control for 30 min at 37°C. Neutralizations with monoclonal anti-IgG, monoclonal anti-IgM and their isotype controls were performed in plasma samples able to detach podocytes from 17 controls, 23 INS proteinuria and 19 INS remission Neutralization efficacy between the 3 groups was not different (χ² test). Differences in neutralization efficacy with antibodies and their isotype controls were tested using the Fisher exact test, *p<0.05, **p<0.01, ***p<0.001.

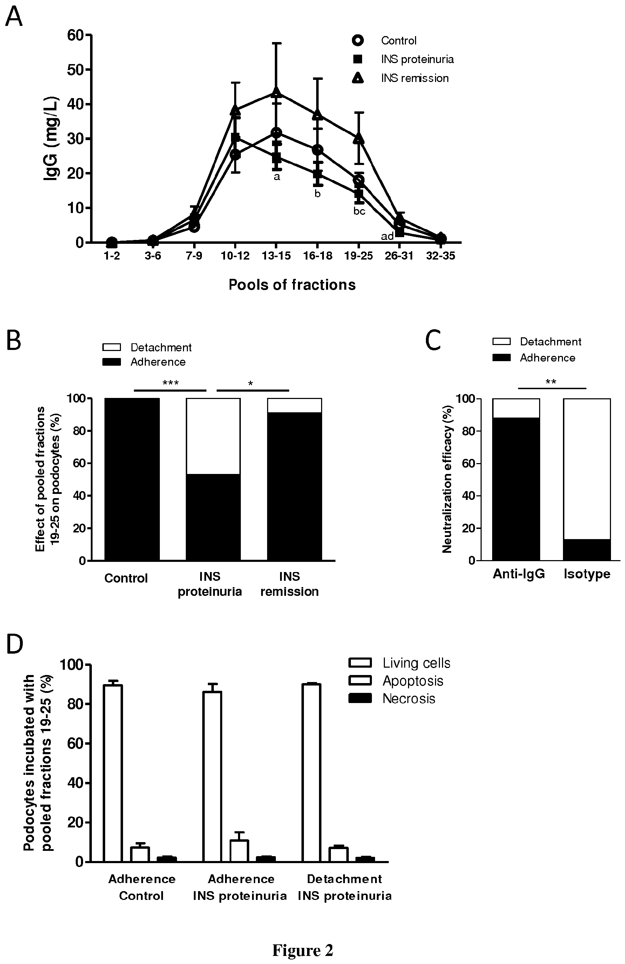

**Figure 2****. Effect of plasma fraction exposure on podocytes.** (A) Measurement of IgGlevels by ELISA in pools of plasma fractions in 27 controls children, 45 INS proteinuria and 17 INS remission; differences between groups were compared with the Kruskal-Wallis test for each pool of fractions. Pools of fractions from INS proteinuria had lower levels of IgG than those from INS remission (a) p<0.01, (b) p<0.05 and than those from controls (c) p<0.01 and (d) p<0.05. (B) Effect of pooled fractions 19-25 from 25 controls, 34 INS proteinuria and 11 INS remission after 2 h of incubation on podocytes. Differences between groups were compared by χ2 test; * p<0.05, *** p<0.001. (C) Neutralization efficacy of 11 pooled fractions 19-25 exposure on podocytes for 2h after co-incubation of these pooled fractions 19-25 from INS proteinuria able to detach podocytes with monoclonal anti-IgG or isotype control for 30 min at 37°C. Fisher exact test was performed to compare differences in neutralization efficacy with anti-IgG and it isotype control, **p<0.01. (D) Viability of podocytes was evaluated by flow cytometry in pooled fractions 19-25 from 6 controls and 4 INS proteinuria inducing no effect on podocytes (adherence) and from 6 INS proteinuria inducing detachment of podocytes after 2 h of incubation and then treatment of all conditions with EDTA. No difference in cell viability was found between groups using χ2 test.

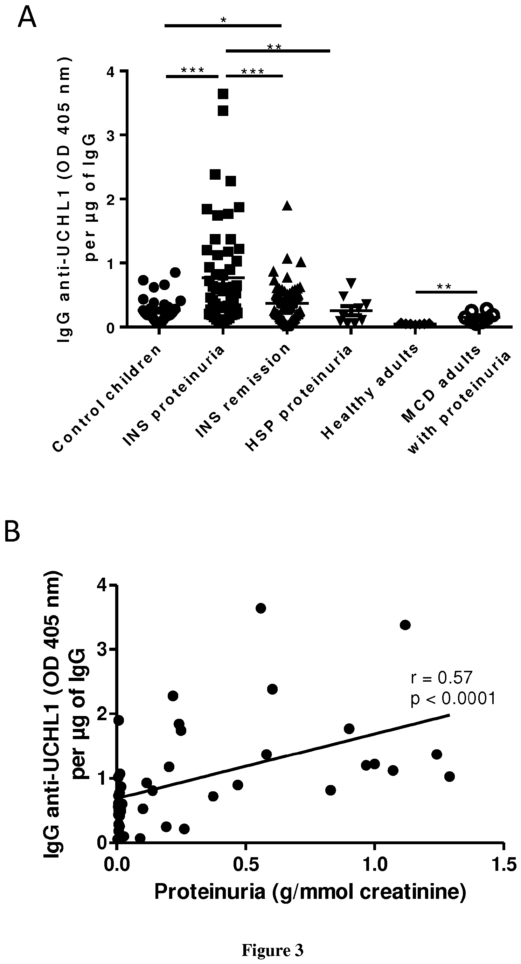

**Figure 3****. Plasma anti-UCHLl IgG level in INS patients and correlation with proteinuria.** (A) Plasma anti-UCHLl IgG were measured by ELISA in 38 control children, 42 INS patients including 58 samples of INS proteinuria and 75 samples of INS remission, 9 proteinuric children with Henoch Schonlein Purpura (HSP), 8 healthy adults and 8 proteinuric adults with minimal change disease (MCD). Differences between groups were evaluated using the non-parametric Kruskal-Wallis test; *p<0.05, **p<0.01, ***p<0.001; p<0.01 between proteinuric patients with INS and HSP; p<0.01 between MCD and healthy adults; p<0.05 between all groups of children compared to healthy adults. (D) Correlation between proteinuria and anti-UCHLl IgG in 43 samples from the 18 INS patients who had one sample with anti-UCHLl IgG per µg of IgG > 0.85 (upper limit of controls) at various stages of the disease (Spearman test). The regression curve intercepted Y axis at 0.69±0.13.

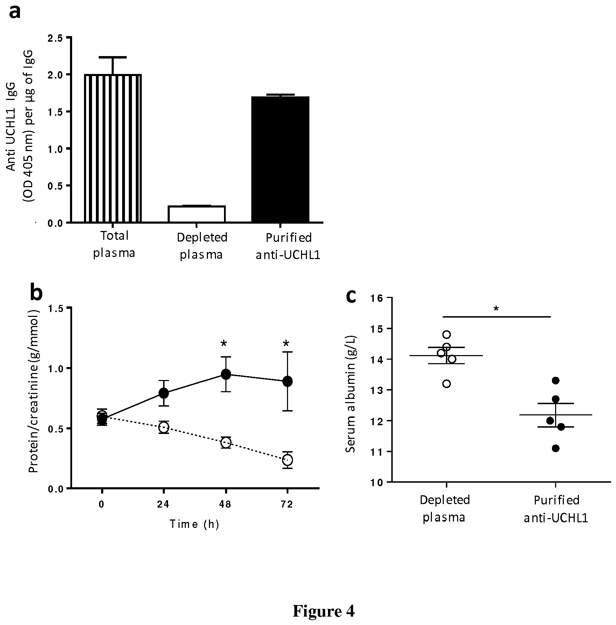

**Figure 4****. Changes of proteinuria and serum albumin after intravenous injection of anti-UCHL1 Abs in mice.**
   **(a)** A plasma sample from a patient with high concentration of circulating anti-UCHLl IgG Abs was submitted to affinity chromatography with a column substituted with recombinant UCHL1. Dosages of anti-UCHL1 IgG Abs showed that the purified fraction retained most of the anti-UCHL1 IgG Abs while the depleted plasma fraction contained an insignificant residual level of anti-UCHLl IgG Abs.
   **(b)** The effect of intravenous injections of the purified fraction of anti-UCHL1 IgG Abs (●) and of the depleted plasma (**O**) in mice were compared. A significant proteinuria was observed at 48 and 72h after intravenous injection (n = 5 mice). *p<0.05
   **(c)** The development of proteinuria was associated with hypoalbuminemia by 72h (C) in mice injected with the purified anti-UCHLl IgG Abs (■) compared to those injected with the depleted plasma (●); n = 5 mice. *p<0.05
**Figure 5****. Renal morphology after intravenous injection of anti-UCHLl Abs in mice.**
   **(a,b)** Kidney slides stained with Masson trichrome: the glomerular morphology was similar in mice injected with depleted plasma **(a)** and purified anti UGHL1 Abs **(b)**
   **(c,d)** Kidney slides with anti-human IgG staining: No IgG deposit was observed in glomeruli from mice injected with depleted plasma **(c)** and purified anti UGHL1 Abs **(d).**
   **(e-h)** Ultramicroscopy revealed major changes in podocyte morphology at different magnification. Mice injected with anti UCHL1 IgG abs exhibited foot process effacement compared to mice injected with depleted plasma where foot process remained perfectly delineated.

### EXAMPLE 1:

### Material & Methods

### Patients and samples

Eighty-five INS patients sampled at various stages of the disease, *i.e.* in relapse and/or in remission and 79 control subjects were prospectively recruited at the Robert Debré Hospital in Paris between 2010 and 2016. The study protocol was approved by the local ethics committee. Pediatric patients were enrolled following written informed consent of the parents. INS children were sampled if they were <18 year-old, at least 1 month from any steroid therapy and either 3 months from immunosuppressive therapy. INS relapse and remission were defined by the French National Authority for Health, respectively a proteinuria/creatininuria ratio >0.25 g/mmol and a hypoalbuminemia <30 g/l, and a proteinuria/creatininuria ratio <0.02 g/mmol and albuminemia >30 g/l. Due to ethical reasons in pediatric, biopsies were not performed in children <11 year-old and in adolescents when they were steroid sensitive and had no complication of INS. It is universally admitted that patients with steroid sensitive nephrotic syndrome commonly display minimal change disease at renal histology. In our series of 85 INS patients with steroid sensitive nephrotic syndrome, only 7 patients had renal histology that consistently showed minimal change disease (MCD). Control subjects were <18 year-old, had neither proteinuria nor a history of nephrotic syndrome, and they had never received steroids or immunosuppressive therapy. Additional patients with others nephropathies and healthy adults were included to validate the identified antibody in the last experiment of our study. Nine Henoch Scholein Purpura (HSP) proteinuric children were included at the Robert Debré Hospital, 8 proteinuric adults suffering from MCD and 8 healthy adult were included at the Xavier Bichat Hospital. Plasma from EDTA venous peripheral blood were collected from patients and controls matched by age and gender and were stored at -80°C until experiments.

### Plasma fractionation

Some plasma aliquots of 500 µL were fractionated by size-exclusion chromatography through a Superdex 200 HR10/30 column (GE Healthcare Life Sciences, Velizy-Villacoublay, France) connected to a High Performance Liquid Chromatography (HPLC) AKTA-basic automated liquid chromatography system (GE Healthcare Life Sciences) and plasma fractions eluted in phosphate buffer saline (PBS) were stored at -20°C until use.

### Podocyte cell culture

The adherent immortalized human podocyte cell line AB8/13 transfected with SV40 retrovirus was proliferated under permissive conditions (33°C, 5% CO2) and was then differentiated under non-permissive conditions (37°C, 5% CO2) as previously described. Podocytes were routinely cultured in RPMI 1640 Glutamax culture medium (Life technologies, Saint-Aubin, France) supplemented with 10 % fetal calf serum (FCS, HyClone, GE Healthcare Life Sciences, Velizy-Villacoublay, France), 1 % penicillin-streptomycin (Sigma-Aldrich) and 1% insulin transferrin selenium (Life Technologies, Villebon sur Yvette, France). Podocytes were used for experimentations after 14 days at 37°C and 1 day of FCS starvation.

### Incubation of total plasma and plasma fractions with podocytes

Human podocytes cultured on uncoated 24-well plates (CytoOne®, Starlab, Orsay, France) were exposed to 2%, 5%, 9%, 13%, 16% and 20% of filtered plasma or PBS or FCS diluted in culture medium during 2 h. Plasma were also neutralized with monoclonal antibodies against IgG (human anti-human IgG Fc, clone 6864, Bio-Rad AbD Serotec Ltd, Oxford, UK) or IgM (mouse anti-human IgM, clone M15/8, Bio-Rad AbD Serotec Ltd), or their irrelevant isotype controls (HuCAL Fab-dHLX-MH negative control antibody from Bio-Rad AbD Serotec Ltd and murine IgG1, clone 320 produced in our lab, respectively) for 30 min at 37°C and then exposed at 13% in culture medium of podocytes during 2 h. Human podocytes cultured on collagen type 1 (0.2 mg/mL, Institut de Biotechnologies Jacques Boy, Reims, France) coated Nunc™ Lab-Tek® 4-chamber slides (ThermoFisher Scientific, Villebon sur Yvette, France) were exposed to 50% of plasma fractions diluted in culture medium during 2 h. Plasma fractions were neutralized with monoclonal antibodies against IgG (human anti-human IgG Fc, clone 6864, Bio-Rad AbD Serotec Ltd) or it irrelevant isotype control (HuCAL Fab-dHLX-MH negative control antibody, Bio-Rad AbD Serotec Ltd) for 30 min at 37°C and then exposed at 50% in culture medium of podocytes during 2 h. After 2 h of total plasma or plasma fraction incubation, podocyte morphology was evaluated by light microscopy (Leica DM-IRB, Leica Microsystems, Nanterre, France).

### Podocyte viability after plasma fraction incubation

After stimulation with plasma fractions, detached and adherent podocytes were incubated with 0.01 M EDTA for 3 min at 37°C, 5% CO2, then washed twice with cold buffer from fluorescein isothiocyanate (FITC) Annexin V Apoptosis Detection Kit I (BD Biosciences, Le Pont de Claix, France), followed by an incubation with annexin V coupled to FITC and propidium iodide for 15 min at room temperature. Then podocytes were collected using BD FACSDiva Software by flow cytometry (FACSCantoII, BD Biosciences). Results were analyzed using the FlowJo software (Ashland, OR, USA) to determine the podocyte viability.

### Double immunoprecipitation and mass spectrometry

Plasma fractions of interest from 3 patients sampled both in relapse and in remission and from 3 controls matched in age and sex were immunoprecipitated for 1 h at room temperature with monoclonal human anti-human IgG Fc, (clone 6864, Bio-Rad AbD Serotec Ltd) or with it irrelevant isotype control (HuCAL Fab-dHLX-MH negative control antibody, Bio-Rad AbD Serotec Ltd), both coupled to CNBr-activated Sepharose 4B beads (GE Healthcare Life Sciences), then washed 6 times with PBS-Tween 20 0.05%, and incubated with podocyte solubilized in radio-immunoprecipitation assay lysis buffer containing protease and phosphatase inhibitor cocktails (all from Sigma-Aldrich) for 1 h at room temperature before 2 last washing with PBS-Tween 20 0.05%. Beads coupled with IgG or with irrelevant isotype control from plasma fractions that have targeted podocyte proteins during double immunoprecipation were digested overnight with 500 ng of trypsin (Sequence Grade Trypsin, Promega, Charbonnières-les-Bains, France) for 16 h at room temperature. Enzymatic activity was stopped by addition of formic acid (Sigma Aldrich) to a final concentration of 3%.The resulting peptides were separated by a method that couples liquid chromatography with tandem mass spectrometry. Data were first processed with Proteome Discoverer 1.4 software (Thermo Fisher Scientific) coupled to an inhouse Mascot search server (Matrix Science, 2.4.1. version, Boston, MA) using UniProtKB/Swiss-Prot database and the following parameters: human species, mass tolerance of 7 ppm for precursor ions or 0.5 Da for fragments, and two missed cleavage sites. Partial chemical modifications such as oxidation, phosphorylation, acetylation, deamidation and glutathionylation, were taken into consideration for the queries. False discovery rate was estimated using a reversed database search approach. The relevant podocyte proteins targeted by IgG from plasma fractions of patients and controls were identified after exclusion of proteins also immunoprecipated by isotype control in all groups. A qualitative analysis of these results with X² test (p-value ≤0.05) was performed to show if identified podocyte proteins were the target or not of IgG from the plasma fractions by comparison of the three groups. Then the relative abundance of each protein identified was estimated by label-free quantification using the Progenesis QI software (Nonlinear Dynamics Ltd, 4.1 version, Newcastle, UK) using the following procedure: chromatogram alignment, peptide abundance normalization, statistical analyses of features, and peptides identification using the Mascot server. A decoy search was performed and the significance threshold was fixed to 0.05. Peptides with an ion score less than 15 were rejected. Conflicts for the identification of some peptides were resolved manually. Peptide features with ANOVA (analysis of variance) p-value ≤0.05 between groups were selected as identified podocyte proteins targeted by IgG from plasma fractions of the different groups.

### Identification of one relevant IgG directed against identified targeted podocyte protein

Human podocytes cultured on uncoated 24-well plates (CytoOne®, Starlab, Orsay, France) were exposed during 2 h to increasing concentrations (2.5 to 40 µg/mL) of commercial IgG directed against 5 relevant podocyte proteins identified after mass spectrometry analysis including rabbit IgG anti-human UCHL1 (Cusabio, Wuhan, P.R. China), or rabbit IgG anti-human ERLIN2 (LSBIO, Seattle, WA, USA), or rabbit anti-human NAMPT (Raybiotech, Norcross, GA, USA) or rabbit IgG anti-human SND1 (LSBio), or mouse IgG anti-TCP 1 alpha (Novusbio, Littleton CO, USA). Localization of UCHL1 in podocytes was performed in adherent podocytes incubated with 0.01 M EDTA for 3 min at 37°C, 5% CO2, then washed with PBS, bovine serum albumin (BSA) 2%, Azide 0.01%. Extracellular UCHL1 staining of podocytes was performed with a rabbit anti-UCHLl coupled to FITC (1/50 dilution, Cusabio) or isotype control coupled to Alexa Fluor 488 (Life technologies) and washed twice before analysis. Intracellular UCHL1 staining was performed with the Cytofix/Cytoperm kit (BD Biosciences) using antibodies described above. Stained podocytes were collected with BD FACSDiva Software by flow cytometry (FACSCantoII). Results were analyzed using the FlowJo software. Podocytes were allowed to adhere on fibronectin-coated coverslips. Cells were fixed with 4% paraformaldehyde in PBS for 15 min at 37°C, then permeabilized in PBS with 0.2% saponin and 0.1% Bovin Serum Albumin (BSA) for 20 min at room temperature, stained with rabbit IgG anti-UCHLl (1/15 dilution) vs mouse anti-ERAP2 (1/200 dilution, clone 3F5, Bio-Techne Ltd., Abingdon, UK) or mouse anti-IRAP (1/100 dilution, clone 3E1, Cell Signaling Technology) or mouse anti-gm130 (1/200 dilution, clone 35/GM130, BD Biosciences) for 1h. After washing in PBS with 0.2% saponin and 0 0.1% BSA podocytes were stained with goat anti-rabbit coupled with Alexa Fluor 488, anti-mouse IgG coupled with Alexa Fluor 568, phalloidin coupled with Alexa Fluor 647 (all diluted at 1/50) and Dapi at 1 µg/mL (all reagents from Life Technologies) for 1h. After washing and mounting, coverslips were observed on a Leica SP8 confocal microscope (Leica Microsystems, Nanterre, France) and analyzed with Image J software.

Then podocytes were incubated with commercial IgG anti-UCHLl at 40 µg/mL preincubated for 30 min at 37°C with increasing concentrations (60, 125, 250 and 500 µg/mL) of albumin and recombinant UCHL1 produced and purified in our lab. Briefly, human UCHL1 DNA sequence was inserted in plasmid pET-28b (+) (Novagen, Millipore SAS, Molsheim, France), then recombinant UCHL1 was produced in *E. coli* T7 express LysY system (New England Biolabs, Evry France) and purified in HiTrap™ TALON crude column, 5 mL Superflow connected to an ÄKTA start chromatography system (both from GE Healthcare Life Sciences). Plasma fractions of interest were also preincubated with 250 and 500 µg/mL of recombinant UCHL1 during 30 min at 37°C then incubated on podocytes. After 2 h of incubation, podocytes morphology was evaluated by light microscopy (Leica DM-IRB).

### Measurement of immunoglobulins and autoantibodies by ELISA

Concentrations of IgG and IgM in total plasma and in plasma fractions were assessed by ELISA using Bethyl kits (Bethyl Laboratories, Montgomery, Texas, USA). Concentrations of IgG and IgM anti-UCHLl in plasma were assessed by ELISA. Microtitration plates were coated with 5 µg/mL of recombinant UCHL1 (Cusabio) in PBS for 2h at room temperature. After washing with PBS-Tween 20 0.05%, the blocking solution (PBS-BSA 1%) was added for 1h at room temperature. Diluted plasma (1/100 and 1/200 in PBS-BSA 1% for measurement of IgG and IgM respectively) and undiluted urines were incubated over night at 4°C. After washing with PBS-Tween 20 0.05%, the detection of antibodies against UCHL1 was performed using a rabbit anti-human IgG (H+L) or a goat anti-human IgM, both coupled with alkaline phosphatase (1/2000, Southern Biotech, Birmingham, AL, USA) and substrate (SIGMAFAST p-nitrophenyl phosphate tablets; Sigma-Aldrich, Saint-Louis, Missouri, USA). Positive control was a sheep IgG anti-human/mouse/rat anti-UCHLl (R&D Systems Europe, Lille, France) detected using a rabbit anti-sheep IgG (H+L) coupled with alkaline phosphatase (Southern Biotech, Birmingham, AL, USA). Plates were read at 405 nm in an Infinite M200 (Tecan, Manndorf, Switzerland).

### Statistical analyses

For all the data except those of proteomics, qualitative data were first analyzed using the Chi2-test and if significant, by Fisher's exact test. Spearman's rank correlation test was used to assess the correlation between 2 variables. Quantitative data were first analyzed by the non parametric Kruskal-Wallis test and if significant, the Mann-Whitney test was applied for between-group analyses. P < 0.05 was considered statistically significant. Results are presented as mean ± s.e.m. Statistical analyses were performed using the GraphPad Prism 5.0 software (La Jolla, CA, USA).

### Results:

### Population characteristics

Table 1 reports characteristics of the 85 INS patients and the 76 controls. The 2 groups were not different for age and gender distributions. Controls consisted of patients with non proteinuric renal diseases. Patients were systematically sampled at distance of any treatment by prednisone or immunosuppressant drugs (Table 2). Biological findings of patients at sampling during proteinuria (n = 84 samples) and remission (n = 86) are provided in Table 2.

### Retraction and detachment of cultured podocytes upon total plasma exposure

Adherent cultured podocytes (Figure 1A) were exposed to increasing concentrations of plasma from controls and INS patients in relapse and remission that induced significant morphological changes in a dose dependent manner in all groups (Table 3). With concentrations below 10% of plasma, podocytes remained attached on the plastic surface but displayed morphological changes marked by a foot process retraction (Figure 1B) in 20 to 40% of controls and below 20% of INS patients. Detachment of podocytes was observed when the plasma concentration was increased over 13% (Figure 1C). Detachment occurred immediately after exposure with a majority of plasma. A delay of 15 minutes was observed with the remaining plasma and detachment was systematically preceded by foot process retraction. Control experiments showed that either phosphate buffer saline (PBS) 20% or foetal calf serum (FCS) 20% in culture medium had no effect on podocytes (Figure 1D and 1E). After washing in fresh culture medium with 10% FCS, detached podocytes were also able to stick again on the plastic surface. Plasma that detached podocytes were previously incubated with either monoclonal anti-IgG or anti-IgM antibodies or irrelevant isotype controls to test the hypothesis of the involvement of immunoglobulins in detachment. Anti-IgG antibodies neutralized the detachment of podocytes (Figure IF) by the plasma from 50 to 60% of patients and controls, opposite to inefficient neutralization with isotype control (p<0.0001, Figure 1G). By contrast, anti-IgM antibodies and it isotype control had no significant effect on detachment by the plasma (p=0.18, Figure 1H). The direct contact of total plasma had finally a dose-dependent deleterious effect on adhesion of podocytes that was prevented by anti-IgG neutralization.

### Detachment of cultured podocytes by fractions of plasma

While the effect of total plasma on podocytes did not showed any statistical differences between controls and INS patients, plasma were fractionated using high performance liquid chromatography by size exclusion. Thirty five plasma fractions (1 to 35) were collected. IgG were detected at various levels in all tested pooled fractions (Figure 2A). Lowest levels of IgG were detected in many pools of fractions (13 to 31) from INS proteinuria compared to INS remission and controls (Figure 2A), as also shown in total plasma of our cohort (Table 2). Neither pools of fractions below 18 or over 26 had no detectable effect on podocytes adhesion. By contrast, a significant detachment of podocyte was observed with 47% of pooled fractions 19-25 from INS proteinuria whereas the same fractions from 25 controls and 91% from those of INS patients in remission had no effect on podocyte adhesion (p<0.0001, Figure 2B). After neutralization with monoclonal anti-IgG of fractions from patients with proteinuria that previously detach the cells, podocytes detachment was avoided in 88% of them (Figure 2C). Previous incubation of the same plasma fractions with an irrelevant isotype control neutralized only 13% of them (p<0.0001). After washing in fresh culture media, podocytes detached by fraction 19-25 were still able to stick again on the plastic surface. Consistently, 90% of detached podocytes by plasma fraction 19-25 from INS patients with proteinuria have a normal viability, as they displayed few features of apoptosis or necrosis (Figure 2D).

### Identification of a target podocyte protein to patient antibodies

The neutralization of plasma fractions 19-25 from INS patients with proteinuria by anti-IgG antibodies suggested that those plasma fractions contained antibodies targeting a specific podocyte protein. Consequently, we performed sequential immunoprecipitations, firstly for the capture of IgG contained in the plasma fractions 19-25 from 3 control children and 3 INS patients at proteinuria and remission, and secondly by the immunoadsorption of podocyte lysates on those captured IgG. The obtained IgG-podocyte protein complexes were digested by trypsin and analyzed using mass spectrometry. Two working lists of candidate podocyte proteins targeted by IgG from plasma fractions were generated.

Among the 1450 different proteins identified by qualitative analysis using Proteome Discoverer, only 29 podocyte proteins were differentially targeted by IgG of plasma fractions from the 3 control and the 3 INS patients (p<0.05). Three proteins including 2 ubiquitous proteins, i.e. cyclin-dependent kinase 1 and eukaryotic translation initiation factor 3 subunit E, and one specific protein of the podocyte, the ubiquitin carboxyl- terminal hydrolase isozyme L1 (UCHL1), were only targeted by IgG from patients with proteinuria (p<0.01). Consequently, UCHL1 was then explored as a potential candidate podocyte target in INS proteinuria.

Moreover, among the 453 different proteins identified by Progenesis QI using quantitative analysis, only 17 podocyte proteins were differentially targeted by IgG of plasma fractions from the 3 controls and the 3 INS patients (p≤0.05). Eleven podocyte proteins were highly targeted by IgG from INS proteinuria compared to others groups and more investigations were conducted in the 4 proteins showing the higher fold change between groups, including erlin-2, T-complex protein 1 subunit alpha (TCP1 alpha), staphylococcal nuclease domain-containing protein 1 (SND1, all these proteins were also significantly different when targeted by IgG of the 3 groups in qualitative analysis in Supplemental Table 1, p<0.043) and nicotinamide phosphoribosyltransferase (NAMPT). Polyclonal commercial antibodies directed against the 5 candidate proteins were tested on podocytes and only one commercial anti-UCHL1 IgG was able to detach podocytes. Consistently UCHL1 showed a minor expression of the protein at the podocyte surface, but the main expression was inside the podocyte and was precisely localized in the endoplasmic reticulum of podocytes. The specificity of anti-UCHL1 IgG was also controlled by the neutralization of podocyte detachment when the antibody was previously incubated with the recombinant protein UCHL1, whereas albumin was not able to neutralize this antibody . As a control experiment, recombinant protein UCHL1 and albumin had no effect on podocyte morphology. Preincubation with recombinant protein UCHL1 can neutralize the plasma fraction of interest (19-25) from INS proteinuria, which were able to detach podocytes.

### Increase of plasma anti-UCHL1 IgG in INS patients with proteinuria

A specific homemade ELISA was elaborated to detect anti-UCHLl IgG in the plasma of different controls and patients. The concentration of anti-UCHLl IgG was significantly increased in INS patients during proteinuria compared to control children and INS patients in remission (Figure 3A). In addition, 9 proteinuric children with IgA nephropathy related to Henoch-Schoenlein Purpura, 8 healthy adults and 8 adults with minimal change disease (MCD) showed very low plasma level of anti-UCHLl IgG. Then, we selected the 18 INS patients with proteinuria, *i.e.* 42% of the tested patients that had a plasma level of anti- UCHL1 IgG over the highest level of controls. For those patients, 43 samples at various stages of the disease were available during other flares and remission phases. In those samples, the level of anti-UCHL1 IgG was significantly correlated to the level of proteinuria (Figure 3B).

### Discussion:

While numerous clinical facts including the efficacy of drugs targeting the B cells and the association of memory B cells with relapses, the involvement of IgG might be reconsidered in INS. Based on a cellular assay of podocyte detachment in vitro and a proteomic approach, we were able to identify an autoantibody of IgG type in the plasma of patients that targets podocytes. It turns out that the targeted protein is UCHL1, a protein mainly expressed in brain, pancreas and kidney, which controls the degradation of proteins in the proteasome through the regulation of ubiquitination.

A first step of our work was to study the effect of different concentrations of total plasma on cultured podocytes. A previous study had showed that the podocyte morphology was disrupted in the presence of 10% plasma from nephrotic patients in culture medium with a retraction of foot process formation.¹² Accordingly, we observed the same morphological changes with plasma concentration below 10%, but with a minority of plasma from INS patients. Paradoxically, up to 50% of control plasma were able to retract the podocytes at a concentration below 10%. Over the concentration of 10%, plasma from INS patients and controls led to podocyte detachment with a prevalence that was proportional to the increase of plasma concentration, suggesting that cultured podocytes are highly sensitive to circulating plasma proteins. Consistently, in vivo podocytes are highly protected from high concentration of plasma proteins due to the retention of plasma proteins in the glycocalyx and the inner layer of the basement membrane.¹³ Interestingly, podocyte retraction in vitro might be understood as a model of minimal change disease while podocyte detachment is already known to be the first cellular step of focal segmental glomerulosclerosis (FSGS). Following the observation that anti-IgG antibodies were able to block the detachment of podocytes by total plasma, we found that cultured podocytes were specifically detached by specific plasma fractions obtained from INS patients with proteinuria. The fact that this fraction contained IgG and that anti-IgG were able to block the detachment of podocyte supported the involvement of specific antibodies. In a continuous effort to identify the specificity of this antibody, we were able to point UCHL1 as a podocyte target. UCHL1 was previously identified in a study of podocyte exosome from urine of healthy individuals.¹⁴

Anti-UCHLl IgG were effectively increased in 42% of the nephrotic patients of our series during proteinuria and their plasma level were correlated with intensity of proteinuria. UCHL1 is involved both in the processing of ubiquitin precursors and of ubiquitinated proteins. UCHL1 is a deubiquitination enzyme that is consequently supposed to protect intracellular proteins from the degradation in the proteasome. UCHL1 is primarily expressed in brain nerve cells and is recognized as a neuronal iconic protein.¹⁵ As a matter of fact, numerous neuronal proteins are expressed in the podocytes. Consistently, Nestin, Neuron-specific enolase, Synaptopodin and UCHL1 play important roles in maintaining the formation of processes in podocytes. 15 The damage of podocytes exposed to adriamycin, a toxic that gives experimental nephrotic syndrome in rats, correlated in a time manner with a down expression of those neuronal proteins resulting in the retraction of the cytoskeleton and changes in cell morphology. UCH-L1 has been associated with irreversible FSGS.¹⁶

Expression of UCHL1 in podocytes has been reported in renal diseases with massive proteinuria.17 UCHL1 was uniformly highly expressed in primary membranous nephritis, while mostly absent in MCD and highly heterogeneous in FSGS which is known to result from different pathophysiological causes.¹⁷ Interestingly, the balance of the proteasome is critical for the stability of podocytes. Rac1 activation in podocytes induces rapid foot process effacement and proteinuria via podocine and nephrin degradation in the proteasomal pathway.¹⁸ β-catenin plays a critical role in mediating podocyte injury/dysfunction in proteinuric kidney disease via the loss of podocyte-specific nephrin, podocalyxin, and synaptopodin in the podocyte due to protein degradation through the ubiquitin proteasomal pathway.¹⁹

This is undoubtful that circulating anti-UCHLl IgG can be physiologically internalized into the podocyte through the neonatal Fc receptor as any antibodies that cross the endothelial layer and the glomerular basement membrane. The transcytosis of antibodies to the urinary chamber ogf the glomerula has been shown to avoid the accumulation of antibodies in the subpodocyte space.²⁰ One appealing hypothesis is that intracellular UCHL1 might be accidentally redirected in the urinary space with anti-UCHLl IgG leading to a podocyte depletion in UCHL1. Consistently, it is supposed that podocyte depletion in UCHL1 activate cytoskeleton protein degradation in the podocyte due to the lack of de-ubiquitination and lead to proteinuria and foot process effacement. As a matter of fact, a lot of podocyte specific proteins (podocin, synaptopodin, nephrin and galectin-1) have a decreased expression during the phase of proteinuria.^{12, 21} Moreover, the level of proteinuria has been inversely correlated to the level of nephrin expression in the podocyte.²² This mechanism also fits with the complete reversibility of the disease via a rapid restoration of podocyte when IgG anti- UCHL1 are cleared from the plasma after a treatment with steroids or immunosuppressive drugs.

In conclusion, a set of INS patients display a significant plasma level of anti-UCHL1 IgG that target the podocytes. Based on the correlation between the plasma level of anti UCHL1 IgG and proteinuria, we suggest that anti UCHL1 IgG plays a central role in the development of massive proteinuria.

### TABLES:

**Table 1. General characteristics of the patients and controls.**

| | Controls* | INS patients | P |
|---|---|---|---|
| *Demographics* | | | |
| Number of patients (n) | 76 | 85 | N/A |
| Gender ratio (male/female) | 49/27 | 57/29 | 0,52 |
| Age (years) | 8,9 (6,4-12,9) | 9.5(6.3-13.1) | 0,51 |

| *Disease characteristics* | | | |
|---|---|---|---|
| Age at onset (years) | N/A | 3.7(2.3-6.2) | 0,99 |
| First flare (n) | N/A | 25 | < 0.0001 |
| Steroid sensitivity (n) | N/A | 12 | 0,25 |
| Steroid dependency (n) | N/A | 48 | < 0.0001 |

| *History of previous therapies* | | | |
|---|---|---|---|
| None (n) | 76 | 25 | |
| Prednisone (n) | 0 | 60 | |
| Mycophenolate mofetil (n) | 0 | 29 | |
| Anti-calcineurin inhibitors (n) | 0 | 23 | |
| Rituximab (n) | 0 | 26 | |

| | | | |
|---|---|---|---|
| *Controls included 41 congenital anomalies of the kidney and the urinary tract, 13 tubular diseases, 6 isolated hematuria, 5 ciliopathies, 5 lithiasis, 5 miscellaneous kidney diseases and 4 healthy controls after systematic family investigation. INS = idiopathic nephritic syndrome; n.a. = not applicable. Data are expressed as median (interquartile range). The 2 groups of patients were equal in term of gender (p = 0.86) and age (p = 0.92). | | | |

**Table 2. Clinical and biological characteristics of patients at sampling**

| | controls | INS proteinuria | INS remission | P |
|---|---|---|---|---|
| Number of samples | 76 | 84 | 86 | |
| Number of relapses since INS onset | N/A | 4 (0-7) | 4 (2-7) | 0,17 |
| Relapse-free period in INS (months) | | | | |
| Steroid free period in INS (months) | N/A | 14,5 (6,3-26,6) | 11,2 (5,6-19,8) | 0,15 |
| Immunosuppressant free period in INS (months) | N/A | 10,9 (6,0-22,2) | 9,2 (4,8-23,8) | 0,86 |
| Proteinuria (g/mmol creatinine) | 0,01 (0,00-0,016)^{a} | 0,365 (0,196-0,971)^{c} | 0,009 (0,006-0,165)^{a} | < 0.0001 |
| Serum albumin (g/L) | 44 (42-45)^{a} | 26 (12-34)^{C} | 42(41-46)^{a} | < 0.0001 |
| eGFR (mL/min/1.73m²) | 105 (91-126)a | 128 (114-147)bd | 122 (103-135)b | < 0.0001 |
| Serum creatinine (µmol/L) | 46 (33-61)a | 37 (29-44)bd | 39 (35-54)ab | 0,005 |
| Plasma IgG (g/L) | 6,9 (4,4-10,2)a | 4,2 (2,0-6,6)c | 6,5 (4,8-9,5)a | < 0,0001 |
| Plasma IgM (g/L) | 0,7 (0,5-1,0)a | 1,0(0,6-1,5)bc | 0,8 (0,5-1,3)a | 0,01 |

| | | | | |
|---|---|---|---|---|
| Data are expressed as median (interquartile range). INS = idiopathic nephritic syndrome including 85 patients with one or more samples at 2 stages of the disease: proteinuria and remission. eGFR = estimated glomerular filtration rate for the children using the revised Schwartz formula (k-value 50413) updated in 2009 (ref); N/A = not applicable. When superscript letters are different (a, b, c, d), medians showed significant difference between groups, P<0.01. | | | | |

### EXAMPLE 2:

### Material & Methods

### Injections of anti-UCHLl Abs to mice

Anti-UCHLl Abs were purified from 8 ml plasma from a patient with high concentration of plasma anti-UCHLl IgG Abs using the AminoLink Plus Immobilization Kit (Thermo Scientific) and concentrated using Amicon Ultra-15 centrifugal filters with a molecular cut-off of 100 kDa to a final volume of 1 ml. Five male BALB/c mice were then intravenously injected once with an adjusted volume containing 200 µg of affinity-purified anti-UCHL1 Abs. The remaining depleted serum was concentrated using Amicon Ultra-15 centrifugal filters with a molecular cut-off of 100 kDa. Five male BALB/c mice were then injected intravenously with an adjusted volume containing 200 µg of total IgG. Development of proteinuria was monitored using metabolic cages every 24h for 72h. Kidneys at 72h were collected, one fixed with 4% paraformaldehyde for Masson Trichrome staining and one frozen in OCT compound for immunohistochemistry with anti-human IgG coupled with biotin (Southern Biotech).

### Results

### Induction of proteinuria in mice by injection of patient anti-UCHLl IgG Abs

Human anti-UCHLl IgG Abs purified by affinity chromatography was prepared from an INS patient at relapse with high plasma levels of anti-UCHLl IgG Abs (Figure 4a). Compared to mice injected with the depleted plasma from the same patient, mice injected with anti-UCHLl IgG Abs exhibited a significantly higher proteinuria and lower serum albumin after 72h (Figure 4b,c). Histology examination of the kidneys harvested in sacrificed animals at 72h showed minor morphological changes and no IgG deposits in animals injected with anti-UCHL1 IgG Abs compared to those injected with PBS and depleted plasma (Figure 5 a-d). Ultramicroscopy showed foot process effacement in mice injected with patient's anti-UCHLl IgG Abs while mice injected with the depleted plasma displayed foot processes with a normal shape.

### REFERENCES:

Throughout this application, various references describe the state of the art to which this disclosure pertains.
1. Eddy AA, Symons JM: Nephrotic syndrome in childhood. Lancet 362: 629-39, 2003
2. Dossier C, Jamin A, Deschenes G: Idiopathic Nephrotic Syndrome: The EBV hypothesis. Pediatr Res, 2016
3. Davin JC: The glomerular permeability factors in idiopathic nephrotic syndrome. Pediatr Nephrol 31: 207-15, 2016
4. Cattran DC, Alexopoulos E, Heering P, Hoyer PF, Johnston A, Meyrier A, Ponticelli C, Saito T, Choukroun G, Nachman P, Praga M, Yoshikawa N: Cyclosporin in idiopathic glomerular disease associated with the nephrotic syndrome : workshop recommendations. Kidney Int 72: 1429-47, 2007
5. Traitanon O, Mathew JM, La Monica G, Xu L, Mas V, Gallon L: Differential Effects of Tacrolimus versus Sirolimus on the Proliferation, Activation and Differentiation of Human B Cells. PLoS One 10: e0129658, 2015
6. Bagga A, Hari P, Moudgil A, Jordan SC: Mycophenolate mofetil and prednisolone therapy in children with steroid-dependent nephrotic syndrome. Am J Kidney Dis 42: 1114-20, 2003
7. Allison AC, Eugui EM: Mycophenolate mofetil and its mechanisms of action. Immunopharmacology 47: 85-118, 2000
8. Iijima K, Sako M, Nozu K, Mori R, Tuchida N, Kamei K, Miura K, Aya K, Nakanishi K, Ohtomo Y, Takahashi S, Tanaka R, Kaito H, Nakamura H, Ishikura K, Ito S, Ohashi Y: Rituximab for childhood-onset, complicated, frequently relapsing nephrotic syndrome or steroid-dependent nephrotic syndrome: a multicentre, double-blind, randomised, placebo-controlled trial. Lancet 384: 1273-81, 2014
9. Colucci M, Carsetti R, Cascioli S, Casiraghi F, Perna A, Rava L, Ruggiero B, Emma F, Vivarelli M: B Cell Reconstitution after Rituximab Treatment in Idiopathic Nephrotic Syndrome. JAm Soc Nephrol 27: 1811-22, 2016
10. Dantal J, Bigot E, Bogers W, Testa A, Kriaa F, Jacques Y, Hurault de Ligny B, Niaudet P, Charpentier B, Soulillou JP: Effect of plasma protein adsorption on protein excretion in kidney-transplant recipients with recurrent nephrotic syndrome. N Engl J Med 330: 7-14, 1994
11. Dantal J, Godfrin Y, Koll R, Perretto S, Naulet J, Bouhours JF, Soulillou JP: Antihuman immunoglobulin affinity immunoadsorption strongly decreases proteinuria in patients with relapsing nephrotic syndrome. JAm Soc Nephrol 9: 1709-15, 1998
12. Coward RJ, Foster RR, Patton D, Ni L, Lennon R, Bates DO, Harper SJ, Mathieson PW, Saleem MA: Nephrotic plasma alters slit diaphragm-dependent signaling and translocates nephrin, Podocin, and CD2 associated protein in cultured human podocytes. J Am Soc Nephrol 16: 629-37, 2005
13. Satchell S: The role of the glomerular endothelium in albumin handling. Nat Rev Nephrol 9: 717-25, 2013
14. Prunotto M, Farina A, Lane L, Pernin A, Schifferli J, Hochstrasser DF, Lescuyer P, Moll S: Proteomic analysis of podocyte exosome-enriched fraction from normal human urine. J Proteomics 82: 193-229, 2013
15. Sun Y, Zhang H, Hu R, Sun J, Mao X, Zhao Z, Chen Q, Zhang Z: The expression and significance of neuronal iconic proteins in podocytes. PLoS One 9: e93999, 2014
16. Beeken M, Lindenmeyer MT, Blattner SM, Radon V, Oh J, Meyer TN, Hildebrand D, Schluter H, Reinicke AT, Knop JH, Vivekanandan-Giri A, Munster S, Sachs M, Wiech T, Pennathur S, Cohen CD, Kretzler M, Stahl RA, Meyer-Schwesinger C:Alterations in the ubiquitin proteasome system in persistent but not reversible proteinuric diseases. J Am Soc Nephrol25: 2511-25, 2014
17. Meyer-Schwesinger C, Meyer TN, Munster S, Klug P, Saleem M, Helmchen U, Stahl RA: A new role for the neuronal ubiquitin C-terminal hydrolase-Ll (UCH-L1) in podocyte process formation and podocyte injury in human glomerulopathies. J Pathol 217: 452-64, 2009
18. Yu H, Suleiman H, Kim AH, Miner JH, Dani A, Shaw AS, Akilesh S: Rac1 activation in podocytes induces rapid foot process effacement and proteinuria. Mol Cell Biol 33:4755-64, 2013
19. Zhou L, Li Y, He W, Zhou D, Tan RJ, Nie J, Hou FF, Liu Y: Mutual antagonism of Wilms' tumor 1 and beta-catenin dictates podocyte health and disease. J Am Soc Nephrol 26: 677-91, 2015
20. Roopenian DC, Akilesh S: FcRn: the neonatal Fc receptor comes of age. Nat RevImmunol 7: 715-25, 2007
21. Shimizu M, Khoshnoodi J, Akimoto Y, Kawakami H, Hirano H, Higashihara E, Hosoyamada M, Sekine Y, Kurayama R, Kurayama H, Joh K, Hirabayashi J, Kasai K, Tryggvason K, Ito N, Yan K: Expression of galectin-1, a new component of slit diaphragm, is altered in minimal change nephrotic syndrome. Lab Invest 89: 178-95,2009
22. Doublier S, Ruotsalainen V, Salvidio G, Lupia E, Biancone L, Conaldi PG, Reponen P, Tryggvason K, Camussi G: Nephrin redistribution on podocytes is a potential mechanism for proteinuria in patients with primary acquired nephrotic syndrome. Am J Pathol 158: 1723-31, 2001
23. Saleem MA, O'Hare MJ, Reiser J, Coward RJ, Inward CD, Farren T, Xing CY, Ni L, Mathieson PW, Mundel P: A conditionally immortalized human podocyte cell line demonstrating nephrin and podocin expression. JAm Soc Nephrol 13: 630-8, 2002
24. Rouillon J, Zocevic A, Leger T, Garcia C, Camadro JM, Udd B, Wong B, Servais L, Voit T, Svinartchouk F: Proteomics profiling of urine reveals specific titin fragments as biomarkers of Duchenne muscular dystrophy. Neuromuscul Disord 24: 563-73, 2014
25. Schwartz GJ, Munoz A, Schneider MF, Mak RH, Kaskel F, Warady BA, Furth SL: New equations to estimate GFR in children with CKD. J Am Soc Nephrol 20: 629-37, 2009

## Claims

1. An *in vitro* method of determining whether a subject suffers from idiopathic steroid sensitive nephrotic syndrome (INS) comprising i) determining the concentration of plasma anti-UCHL1 IgG in a blood sample obtained from the subject ii) comparing the concentration determined at step i) with a predetermined reference value and iii) concluding that the subject suffer from idiopathic steroid sensitive nephrotic syndrome when the concentration determined at step i) is higher than the predetermined reference value.

2. An *in vitro* method of predicting the risk of relapse in a subject suffering from INS i) comprising determining the concentration of plasma anti-UCHLl IgG in a blood sample obtained from the subject ii) comparing the concentration determined at step i) with a predetermined reference value and iii) concluding that the subject is at risk of relapse when the concentration determined at step i) is higher than the predetermined reference value.

3. The method of claim 1 or 2 wherein the predetermined reference value is the concentration of plasma anti-UCHLl IgG determined in a population of healthy individuals.

4. The method of claim 3 wherein it is concluded that the patient suffers from INS or is at risk of relapse when the concentration of plasma anti-UCHL1 IgG is at least 1.5 fold higher than the concentration determined in a population of healthy individuals.

5. An *in vitro* method of determining whether the subject achieves a response with an immunosuppressive treatment comprising i) determining the concentration of plasma anti-UCHL1 IgG in a blood sample obtained from the subject before the treatment ii) determining the concentration of plasma anti-UCHLl IgG in a sample obtained from the subject after the treatment, iii) comparing the concentration determined at step i) with the concentration determined at step ii) and iv) concluding that the subject achieves a response when the concentration determined at step ii) lower than the concentration determined at step i).

6. The method of claim 5wherein the immunosuppressive agent is selected from the group consisting of 6-mercaptopurine ("6-MP"), cyclophosphamide, mycophenolate, prednisolone, sirolimus, dexamethasone, rapamycin, FK506, mizoribine, azathioprine and tacrolimus.

7. The method of claim 5 wherein the immunosuppressive agent is selected from the group consisting of calcineurin inhibitors, corticosteroids, and B cell depleting agents.

8. The method of claim 7 wherein the B cell depleting agent is rituximab.

9. The method of claim 7 wherein a decrease in the concentration after the treatment compared to the concentration before the treatment of at least 5%, at least 10%, at least 20%, at least 30%, at least 40%, at least 50%, at least 60%, at least 70%, at least 80%, at least 90%, 100% indicates that the subject achieves a response.

10. The method of claim 7 wherein a concentration which returns to the basal concentration indicates that the subject has achieved a response.

11. The method of claim 1 or 2 wherein the detection and quantification of anti-UCHL1 IgG in the sample is performed by ELISA.

12. The method of claim 1 or 2 wherein the detection and quantification of anti-UCHLl IgG is performed with a kit or device comprising at least a UCHL1 protein or fragments thereof; at least one solid support wherein the UCHL1 protein or fragments thereof is deposited on the support and a detection antibody, wherein the detection antibody is specific for the anti-UCHL1 IgG in the sample of the subject and the detection antibody produces a detectable signal.

13. At least one immunosuppressive agent for use in the treatment of idiopathic steroid sensitive nephrotic syndrome (INS) in a subject in need thereof comprising the steps of: i) diagnosing the subject by the method of claim 1 and ii) administering to the said subject a therapeutically effective amount of at least one immunosuppressive agent wherein the immunosuppressive agent is selected from the group consisting of 6-mercaptopurine ("6-MP"), cyclophosphamide, mycophenolate, prednisolone, sirolimus, dexamethasone, rapamycin, FK506, mizoribine, azathioprine, tacrolimus, calcineurin inhibitors, corticosteroids, and B cell depleting agents.

14. The at least one immunosuppressive agent for use according to claim 14 wherein the immunosuppressive agent is rituximab.

## Patentansprüche

1. *In vitro*-Verfahren zum Bestimmen, ob eine Person an einem idiopathischen steroidsensitiven nephrotischen Syndrom (INS) leidet, umfassend i) Bestimmen der Konzentration von Plasma-Anti-UCHL1-IgG in einer von der Person erhaltenen Blutprobe, ii) Vergleichen der bei Schritt i) bestimmten Konzentration mit einem vorbestimmten Referenzwert und iii) Folgern, dass die Person an einem idiopathischen steroidsensitiven nephrotischen Syndrom leidet, wenn die bei Schritt i) bestimmte Konzentration höher als der vorbestimmte Referenzwert ist.

2. *In vitro*-Verfahren zum Vorhersagen des Rezidivrisikos einer an INS leidenden Person, i) umfassend Bestimmen der Konzentration von Plasma-Anti-UCHL1-IgG in einer von der Person erhaltenen Blutprobe, ii) Vergleichen der bei Schritt i) bestimmten Konzentration mit einem vorbestimmten Referenzwert und iii) Folgern, dass ein Rezidivrisiko der Person besteht, wenn die bei Schritt i) bestimmte Konzentration höher als der vorbestimmte Referenzwert ist.

3. Verfahren nach Anspruch 1 oder 2, wobei der vorbestimmte Referenzwert die in einer Population gesunder Personen bestimmte Konzentration von Plasma-Anti-UCHLl-IgG ist.

4. Verfahren nach Anspruch 3 wobei gefolgert wird, dass der Patient an INS leidet oder dass bei diesem ein Rezidivrisiko besteht, wenn die Konzentration von Plasma-Anti-UCHL1-IgG mindestens 1,5-fach höher als die in einer Population gesunder Personen bestimmte Konzentration ist.

5. *In vitro*-Verfahren zum Bestimmen, ob die Person eine Reaktion mit einer immunsuppressiven Behandlung erreicht, umfassend i) Bestimmen der Konzentration von Plasma-Anti-UCHL1-IgG in einer vor der Behandlung von der Person erhaltenen Blutprobe, ii) Bestimmen der Konzentration von Plasma-Anti-UCHL1-IgG in einer nach der Behandlung von der Person erhaltenen Probe, iii) Vergleichen der bei Schritt i) bestimmten Konzentration mit der bei Schritt ii) bestimmten Konzentration und iv) Folgern, dass die Person eine Reaktion erreicht, wenn die bei Schritt ii) bestimmte Konzentration niedriger als die bei Schritt i) bestimmte Konzentration.

6. Verfahren nach Anspruch 5, wobei das Immunsuppressivum ausgewählt ist aus der Gruppe bestehend aus 6-Mercaptopurin ("6-MP"), Cyclophosphamid, Mycophenolat, Prednisolon, Sirolimus, Dexamethason, Rapamycin, FK506, Mizoribin, Azathioprin und Tacrolimus.

7. Verfahren nach Anspruch 5, wobei das Immunsuppressivum ausgewählt ist aus der Gruppe bestehend aus Calcineurininhibitoren, Corticosteroiden und B-Zell-Depletionsmitteln.

8. Verfahren nach Anspruch 7, wobei das B-Zell-Depletionsmittel Rituximab ist.

9. Verfahren nach Anspruch 7, wobei eine Abnahme der Konzentration nach der Behandlung gegenüber der Konzentration vor der Behandlung von mindestens 5 %, mindestens 10 %, mindestens 20 %, mindestens 30 %, mindestens 40 %, mindestens 50 %, mindestens 60 %, mindestens 70 %, mindestens 80 %, mindestens 90 %, 100 % angibt, dass die Person eine Reaktion erreicht.

10. Verfahren nach Anspruch 7, wobei eine Konzentration, die zu der Basalkonzentration zurückkehrt, angibt, dass die Person eine Reaktion erreicht hat.

11. Verfahren nach Anspruch 1 oder 2, wobei der Nachweis und die Quantifizierung von Anti-UCHL1-IgG in der Probe durch ELISA durchgeführt wird.

12. Verfahren nach Anspruch 1 oder 2, wobei der Nachweis und die Quantifizierung von Anti-UCHL1-IgG mit einem Kit oder einer Vorrichtung durchgeführt wird, umfassend mindestens ein UCHL1-Protein oder Fragmente davon, mindestens einen Festkörperträger, wobei das UCHL1-Protein oder Fragmente davon an dem Träger angelagert sind, und einen Nachweisantikörper, wobei der Nachweisantikörper spezifisch für das Anti-UCHL1-IgG in der Probe der Person ist und der Nachweisantikörper ein nachweisbares Signal erzeugt.

13. Mindestens ein Immunsuppressivum zur Verwendung in der Behandlung eines idiopathischen steroidsensitiven nephrotischen Syndroms (INS) bei einer Person, die diese benötigt, umfassend die Schritte: i) Diagnostizieren der Person durch das Verfahren nach Anspruch 1 und ii) Verabreichen, an die Person, einer therapeutisch wirksamen Menge von mindestens einem Immunsuppressivum, wobei das Immunsuppressivum ausgewählt ist aus der Gruppe bestehend aus 6-Mercaptopurin ("6-Mp"), Cyclophosphamid, Mycophenolat, Prednisolon, Sirolimus, Dexamethason, Rapamycin, FK506, Mizoribin, Azathioprin, Tacrolimus, Calcineurininhibitoren, Corticosteroiden und B-Zell-Depletionsmitteln.

14. Mindestens ein Immunsuppressivum zur Verwendung nach Anspruch 14, wobei das Immunsuppressivum Rituximab ist.

## Revendications

1. Procédé *in vitro* pour déterminer si un sujet souffre d'un syndrome néphrotique idiopathique sensible aux stéroïdes (SNI) comprenant les étapes consistant à i) déterminer la concentration d'IgG anti-UCHLl plasmatique dans un échantillon de sang prélevé auprès du sujet, ii) comparer la concentration déterminée à l'étape i) à une valeur de référence prédéterminée et iii) conclure que le sujet souffre d'un syndrome néphrotique idiopathique sensible aux stéroïdes lorsque la concentration déterminée à l'étape i) est supérieure à la valeur de référence prédéterminée.

2. Procédé *in vitro* de prédiction du risque de rechute chez un sujet souffrant du SNI comprenant les étapes consistant à i) déterminer la concentration d'IgG anti-UCHLl plasmatique dans un échantillon de sang prélevé auprès du sujet, ii) comparer la concentration déterminée à l'étape i) à une valeur de référence prédéterminée et iii) conclure que le sujet court un risque de rechute lorsque la concentration déterminée à l'étape i) est supérieure à la valeur de référence prédéterminée.

3. Procédé selon la revendication 1 ou 2, dans lequel la valeur de référence prédéterminée est la concentration d'IgG anti-UCHLl plasmatique déterminée dans une population d'individus sains.

4. Procédé selon la revendication 3, dans lequel il est conclu que le patient souffre de SNI ou court un risque de rechute lorsque la concentration d'IgG anti-UCHLl plasmatique est au moins 1,5 fois supérieure à la concentration déterminée dans une population d'individus sains.

5. Procédé *in vitro* pour déterminer si le sujet obtient une réponse avec un traitement immunosuppresseur comprenant les étapes consistant à i) déterminer la concentration d'IgG anti-UCHLl plasmatique dans un échantillon de sang prélevé auprès du sujet avant le traitement, ii) déterminer la concentration d'IgG anti-UCHLl plasmatique dans un échantillon prélevé auprès du sujet après le traitement, iii) comparer la concentration déterminée à l'étape i) avec la concentration déterminée à l'étape ii) et iv) conclure que le sujet obtient une réponse lorsque la concentration déterminée à l'étape ii) est inférieure à la concentration déterminée à l'étape i).

6. Procédé selon la revendication 5, dans lequel l'agent immunosuppresseur est choisi dans le groupe consistant en 6-mercaptopurine (« 6-MP »), cyclophosphamide, mycophénolate, prednisolone, sirolimus, dexaméthasone, rapamycine, FK506, mizoribine, azathioprine et tacrolimus.

7. Procédé selon la revendication 5, dans lequel l'agent immunosuppresseur est choisi dans le groupe consistant en inhibiteurs de calcineurine, corticostéroïdes et agents de déplétion de cellules B.

8. Procédé selon la revendication 7, dans lequel l'agent de déplétion de cellules B est le rituximab.

9. Procédé selon la revendication 7, dans lequel une diminution de la concentration après le traitement par comparaison à la concentration avant le traitement d'au moins 5 %, d'au moins 10 %, d'au moins 20 %, d'au moins 30 %, d'au moins 40 %, d'au moins 50 %, d'au moins 60 %, d'au moins 70 %, d'au moins 80 %, d'au moins 90 %, de 100 % indique que le sujet obtient une réponse.

10. Procédé selon la revendication 7, dans lequel une concentration qui revient à la concentration basale indique que le sujet a obtenu une réponse.

11. Procédé selon la revendication 1 ou 2, dans lequel la détection et la quantification d'IgG anti-UCHLl dans l'échantillon sont effectuées par ELISA.

12. Procédé selon la revendication 1 ou 2, dans lequel la détection et la quantification d'IgG anti-UCHLl sont effectuées avec un kit ou dispositif comprenant au moins une protéine UCHL1 ou des fragments de celle-ci; au moins un support solide dans lequel la protéine UCHL1 ou des fragments de celle-ci est/sont déposé(s) sur le support et un anticorps de détection, dans lequel l'anticorps de détection est spécifique à l'IgG anti-UCHL1 dans l'échantillon du sujet et l'anticorps de détection produit un signal détectable.

13. Au moins un agent immunosuppresseur à utiliser dans le traitement du syndrome néphrotique idiopathique sensible aux stéroïdes (SNI) chez un sujet qui en a besoin, comprenant les étapes consistant à : i) diagnostiquer le sujet par l'intermédiaire du procédé selon la revendication 1 et ii) administrer audit sujet une quantité thérapeutiquement efficace d'au moins un agent immunosuppresseur dans lequel l'agent immunosuppresseur est choisi dans le groupe consistant en 6-mercaptopurine (« 6-MP »), cyclophosphamide, mycophénolate, prednisolone, sirolimus, dexaméthasone, rapamycine, FK506, mizoribine, azathioprine , tacrolimus, inhibiteurs de calcineurine, corticostéroïdes et agents de déplétion de cellules B.

14. Le au moins un agent immunosuppresseur à utiliser selon la revendication 14, dans lequel l'agent immunosuppresseur est le rituximab.
